# EUROPEAN PATENT SPECIFICATION

(11) **EP 4 214 308 B1**
(45) Date of publication and mention of the grant of the patent: **20.11.2024**
(21) Application number: 21777795.2
(22) Date of filing: 21.09.2021
(51) Int. Cl.: C12N 9/02, C12N 15/90, C12N 9/00, C07K 16/00

(54) **GENERATION OF A HIGH PRODUCING RECOMBINANT CHINESE HAMSTER OVARY CELL LINE FOR THERAPEUTIC PROTEIN PRODUCTION**
ERZEUGUNG EINER HOCHPRODUZIERENDEN REKOMBINANTEN OVARIALZELLLINIE DES CHINESISCHEN HAMSTERS ZUR THERAPEUTISCHEN PROTEINPRODUKTION
GÉNÉRATION D'UNE LIGNÉE CELLULAIRE D'OVAIRE DE HAMSTER CHINOIS RECOMBINANT À HAUT RENDEMENT POUR LA PRODUCTION DE PROTÉINES THÉRAPEUTIQUES

(30) Priority: 21.09.2020 EP 20306068
(43) Date of publication of application: 26.07.2023
(73) Proprietor: Sanofi, 75017 Paris (FR)
(72) Inventor: DUMAS, Bruno Louis, 75008 PARIS (FR); LOUNIS, Mohammed Nabil, 75008 PARIS (FR)
(74) Representative: Lavoix
(86) International application number: PCT/EP2021/075864
(87) International publication number: WO 2022/058603

(56) References cited:
- WO-A1-2016/062837
- WO-A1-2020/012446
- WO-A1-2020/188021
- WO-A2-2020/243642
- US-A1- 2003 166 201

## Description

### Field of the invention

The present invention concerns cell lines and selection markers for protein production.

### Background of the invention

Producing recombinant proteins on an industrial scale requires isolation of clones producing high amounts of recombinant proteins. Introducing heterologous genes into animal host cells and screening for expression of the added genes is a lengthy and complicated process. The process involves the transfection and the selection of clones with stable long-term expression, and the screening for clones having high expression rates for the corresponding recombinant protein.

When generating clones expressing a recombinant protein from expression vectors, host cells are usually transfected with a DNA vector encoding both the protein of interest and the selection marker on the same vector. Such an expression vector thus comprises a selectable marker allowing the selection of clones in which the expression vector is present. Such a selectable marker may also lead to a co-amplification of transfected DNA, thereby allowing the isolation of high-producer clones.

Most selectable markers are either a protein conferring resistance to an antibiotic or other toxic substance or a protein essential to cell survival. Several such selectable markers are known in the art, including e.g.G418, hygromycin, puromycin, zeomycin, dihydrofolate reductase (DHFR), glutamine synthetase (GS) and hypoxanthine-guanine phosphoribosyltransferase (HPRT).

GS is widely used as a selectable marker in the field of industrial recombinant protein production in eukaryotic cells. The GS gene permits the synthesis of glutamine, essential for cell growth, and is inhibited by MSX (L-methionine sulfoximine). In the presence of MSX, only cells expressing higher amount of GS do survive. After appropriate screening it is possible to select cells producing the exogenous proteins.

In previous application WO2016/062837, the inventors developed an expression system based on the use of dehydroorotate dehydrogenase (DHODH) as a selectable marker. DHODH is an enzyme required for pyrimidine synthesis. Compounds which inhibit DHODH therefore inhibit DNA synthesis and hence cell proliferation. This selection marker thus comprises an expression vector encoding DHODH used in combination with a DHODH inhibitor such as leflunomide and teriflunomide.

However, most of the inhibitors used with the above selection markers are toxic. In the case of the DHODH selection marker, teriflunomide is for example a potent immune-suppressor and its handling especially at large scale can be challenging for safety reasons. In the case of the GS selection marker, MSX is a convulsant at high doses and may thus also raise handling issues. In the case of DHFR selection marker, methotrexate is known for displaying hematopoietic and digestive toxicities, thereby also raising handling issues.

Furthermore, for producing complex proteins, such as bispecific or trispecific monoclonal antibodies, it is necessary that a single clone produce several separate recombinant proteins from several separate expression vectors. In such cases, host cells have to be transfected with several DNA vectors, each encoding both a protein of interest and a specific selection marker on the same vector. Each expression vector thus comprises a distinct selectable marker allowing the selection of clones in which the expression vector is present.

Accordingly, there is a need for expression systems enabling the production of several distinct proteins through several distinct selection markers, and where the selection of the proteins of interest-producing clone can be performed without addition of difficult to handle compounds.

The present invention meets this need.

### Summary of the invention

The present invention arises from the design by the inventors of a cell line wherein proteins of interest-producing cells can be selected in a medium devoid of uridine and of glutamine, thanks to the partial or full inactivation of the DHODH gene and of the GS gene in said cell line. This cell line, wherein the DHODH gene and the GS gene are partially or fully inactivated, is typically grown in a medium supplemented with uridine and glutamine, but, when transfected with an expression vector comprising a nucleotide sequence encoding a mammalian DHODH, in particular encoding a mutated mammalian DHODH, and an expression cassette for expressing a first protein of interest, and with an expression vector comprising a nucleotide sequence encoding a mammalian GS and an expression cassette for expression a second protein of interest, the culture medium is typically changed by a culture medium devoid of uridine and of glutamine, thereby selecting the cells producing the first and second proteins of interest.

Such an expression system is particularly advantageous because, by avoiding the use of inhibitors as selection pressure, it increases the viability of the producing cells. The inventors further demonstrated that this decrease in toxicity was associated with a high productivity. Furthermore, such an expression system enables the production of complex recombinant proteins such as bispecific or trispecific monoclonal antibodies.

The present invention thus concerns a cell line comprising:
- an endogenous dehydroorotate dehydrogenase (DHODH) gene which is partially or fully inactivated, and
- an endogenous glutamine synthetase (GS) gene which is partially or fully inactivated.

In a particular embodiment, said cell line is a Chinese Hamster Ovary (CHO) cell line.

In another particular embodiment, the cell line is produced by:
(a1) inactivating the endogenous DHODH gene in a cell comprising an endogenous DHODH gene and an endogenous GS gene, in particular by a gene editing method, such as by a CRISPR-Cas method, such as by a CRISPR-Cas9 method,
(b1) culturing the cell in a culture medium comprising uridine under conditions suitable for generating a cell line in which the endogenous DHODH gene is partially or fully inactivated,
(c1) recovering said cell wherein the endogenous DHODH gene is partially or fully inactivated,
(d1) inactivating the endogenous GS gene in said cell, in particular by a gene editing method, such as by a CRISPR-Cas method, such as by a CRISPR-Cas9 method, and
(e1) culturing said cell in a culture medium comprising glutamine under conditions suitable for generating a cell line in which the endogenous GS gene is partially or fully inactivate.

In an alternative particular embodiment, the cell line is produced by:
(a2) providing a cell comprising an endogenous GS gene, and an endogenous DHODH gene which is partially or fully inactivated,
(b2) inactivating the endogenous GS gene is said cell, in particular by a gene editing method, such as by a CRISPR-Cas method, such as by a CRISPR-Cas9 method, and
(c2) culturing the cell in a culture medium comprising glutamine under conditions suitable for generating a cell line in which the endogenous GS gene is partially or fully inactivated.

In still an alternative particular embodiment, the cell line is produced by:
(a3) providing a cell comprising and endogenous DHODH gene, and an endogenous GS gene which is partially or fully inactivated,
(b3) inactivating the endogenous DHODH gene in said cell, in particular by a gene editing method, such as by a CRISPR-Cas method, such as by a CRISPR-Cas9 method, and
(c3) culturing the cell in a culture medium comprising uridine under conditions suitable for generating a cell line in which the endogenous DHODH gene is partially or fully inactivated.

In a particular embodiment, one or more or all the alleles of the endogenous DHODH gene are partially or fully inactivated, and/or all the alleles of the endogenous GS gene are partially or fully inactivated.

In a further embodiment, said cell line further comprises:
- an expression vector comprising a nucleotide sequence (i) encoding an exogenous mammalian DHODH and at least one expression cassette for expressing a recombinant protein (I), wherein said exogenous DHODH comprises a sequence at least 60% identical to the sequence SEQ ID NO: 2 or to the sequence SEQ ID NO: 4, and
- an expression vector comprising a nucleotide sequence (ii) encoding an exogenous mammalian GS and at least one expression cassette for expressing a recombinant protein (II), wherein said exogenous GS comprises a sequence at least 94.5% identical to the sequence SEQ ID NO: 6 or to the sequence SEQ ID NO: 8.

In a particular embodiment thereof, said nucleotide sequence (i) comprises the sequence of SEQ ID NO: 1 or the sequence of SEQ ID NO: 3.

In another particular embodiment thereof, said nucleotide sequence (ii) comprises the sequence of SEQ ID NO: 5 or the sequence of SEQ ID NO: 7.

In another particular embodiment thereof, said recombinant proteins (I) and (II) are distinct arms of a monoclonal antibody.

In still another particular embodiment thereof, said vectors respectively comprise a first expression cassette suitable for cloning of an antibody light chain, and a second expression cassette suitable for cloning of an antibody heavy chain.

Another object of the invention is an expression system comprising:
(A) the cell line comprising an endogenous DHODH gene which is partially or fully inactivated and an endogenous GS gene which is partially or fully inactivated, as defined above,
(B) an expression vector comprising a nucleotide sequence (i) encoding a mammalian DHODH and at least one expression cassette for expressing a recombinant protein (I), wherein said DHODH comprises a sequence at least 60% identical to the sequence SEQ ID NO: 2 or to the sequence SEQ ID NO: 4, and
(C) an expression vector comprising a nucleotide sequence (ii) encoding a mammalian GS ad at least one expression cassette for expressing a recombinant protein (II), wherein the GS comprises a sequence at least 94.5% identical to the sequence SEQ ID NO: 6 or the sequence SEQ ID NO: 8.

In a particular embodiment, said nucleotide sequence (i) comprises the sequence of SEQ ID NO: 1 or the sequence of SEQ ID NO: 3.

In another particular embodiment, said nucleotide sequence (ii) comprises the sequence of SEQ ID NO: 5 or the sequence of SEQ ID NO: 7.

In another particular embodiment, said recombinant proteins (I) and (II) are distinct arms of a monoclonal antibody.

In still a particular embodiment, said vectors comprise respectively a first expression cassette suitable for cloning of an antibody light chain, and a second expression cassette suitable for cloning of an antibody heavy chain.

The present invention further concerns a kit comprising:
- the cell line as defined above or the expression system as defined above, and
- a culture medium devoid of uridine and of glutamine, in particular further devoid of DHODH inhibitor and of GS inhibitor.

Another object of the invention relates to an *in vitro* method of producing recombinant proteins comprising the steps of:
A)
   a1) providing a cell line as defined above further comprising;
      - an expression vector comprising a nucleotide sequence (i) encoding an exogenous mammalian DHODH and at least one expression cassette for expressing a recombinant protein (I), wherein said exogenous DHODH comprises a sequence at least 60% identical to the sequence SEQ ID NO: 2 or to the sequence SEQ ID NO: 4, and
      - an expression vector comprising a nucleotide sequence (ii) encoding an exogenous mammalian GS and at least one expression cassette for expressing a recombinant protein (II), wherein said exogenous GS comprises a sequence at least 94.5% identical to the sequence SEQ ID NO: 6 or to the sequence SEQ ID NO: 8,
      or
   a2) providing a cell line as defined above, and
   a2') introducing expression vectors as defined above into the cell line provided in step a2);
      or
   a3) providing a cell line comprising an endogenous DHODH gene and an endogenous GS gene,
   a3') partially or fully inactivating the endogenous DHODH gene and the endogenous GS gene in the cell line provided in step a3), and
   a3") introducing expression vectors as defined above into the cell line comprising a partially or fully inactivated endogenous DHODH gene and a partially or fully inactivated GS gene obtained in step a3');
      or
   a4) providing a cell line comprising an endogenous GS gene, and an endogenous DHODH gene which is partially or fully inactivated,
   a4') partially or fully inactivating the endogenous GS gene in the cell line provided in step a4), and
   a4") introducing expression vectors as defined above into the cell line comprising a partially or fully inactivated endogenous DHODH gene and a partially or fully inactivated GS gene obtained in step a4');
      or
   a5) providing a cell comprising an endogenous DHODH gene, and an endogenous GS gene which is partially or fully inactivated,
   a5') partially or fully inactivating the endogenous DHODH gene in the cell line provided in step a5), and
   a5") introducing expression vectors as defined above into the cell line comprising a partially or fully inactivated endogenous DHODH gene and a partially or fully inactivated GS gene obtained in step a5');
B) culturing said cell line under conditions suitable for production of the recombinant proteins; and
C) isolating and/or purifying said recombinant proteins.

In a particular embodiment, said step B) is conducted in a culture medium devoid of uridine and of glutamine, in particular further devoid of DHODH inhibitor and of GS inhibitor.

In another particular embodiment, said method further comprises a step D) of formulating said recombinant proteins into a pharmaceutical composition.

The present invention further concerns the use of a cell line as defined above, an expression system as defined above, or a kit as defined above, for producing recombinant proteins.

In a particular embodiment, the cell line, the expression system or the kit is used in combination with a culture medium devoid of uridine and of glutamine, in particular in the absence of DHODH inhibitor and of GS inhibitor.

### Brief description of the figures

**Figure 1** shows the genomic structure of the human DHODH gene referenced under the Gene ID: 100756632 available on 21 December 2018 on Genebank NCBI.
**Figure 2** shows trispecific antibody production at days 10 and 14 using human DHODH and human GS selection markers and DHODH/GS KO (Knockout) or wild-type CHO cells.

### Detailed description of the invention

### Dihydroorotate dehydrogenase

As used herein, the term "dihydroorotate dehydrogenase" or "DHODH" refers to a polypeptide capable of catalyzing the conversion of dihydroorotate (4,5-dihydroorotic acid or 2,6-dioxo-1 ,3-diazinane-4-carboxylic acid) to orotate (orotic acid or 1,2,3,6-tetrahydro-2,6-dioxo-4-pyrimidinecarboxylic acid), as represented by the following reaction:

(S)-dihydroorotate + O₂ ↔ orotate + H₂O₂

Such a polypeptide is classified under Enzyme Commission (EC) number 1.3.3.1. Polypeptides capable of catalyzing the above reaction exhibit "DHODH activity".

The above reaction is the fourth step in the *de novo* synthesis of uridine monophosphate (rUMP) required for the synthesis of DNA and RNA. Inhibition or inactivation of DHODH thus has the effect of inhibiting DNA and RNA synthesis and hence inhibits cell proliferation.

### Glutamine synthetase

As used herein, the term "glutamine synthetase" or "GS" refers to a polypeptide capable of catalyzing the condensation of glutamate and ammonia to form glutamine, as represented by the following biochemical reaction:

ATP + L-glutamate + NH₃ ↔ ADP + phosphate + L-glutamine

Such a polypeptide is classified under Enzyme Commission (EC) number 6.3.1.2. Polypeptides capable of catalyzing the above reaction exhibit "GS activity".

### Cell line

The present invention concerns a cell line comprising an endogenous dehydroorotate dehydrogenase (DHODH) gene which is partially or fully inactivated and an endogenous glutamine synthetase (GS) gene which is partially or fully inactivated.

The cell line is an eukaryotic cell line, *e.g.* a mammalian cell line such as a Chinese Hamster Ovary (CHO) cell line, a monkey cell line or a human cell line.

In a particular embodiment, the cell line is a CHO cell line.

CHO cell lines are commonly used for industrial protein production, and many CHO cell lines are known to those skilled in the art. For instance, such CHO cell lines include strains which are publicly available from the American Type Culture Collection such as the CHO-K1 cell line (ATCC Number: CCL-61), the CHO-S cell line (marketed for instance by Invitrogen and Gibco), the CHO DP-12 cell line (ATCC Nos. CRL-12444 and 12445) and the CHO 1-15 cell line (ATCC Number CRL-9606). Another cell line suitable for industrial protein production is the CHO 9E4 cell line. The 9E4 cell line was established from a clone of the CHO-K1 cell line through a single cell cloning process. The establishment of the 9E4 cell line is presented more deeply in **Example 1.** The CHO-K1 cell line was obtained by Puck in 1957 and has been deposited at the ATCC under number CCL-61.

Human cells such as HEK293 (ATCC Number CRL-1573), HKB11 (ATCC Number CRL-12568), PER-C6 (Crucell), HT1080 (ATCC Number CRL-121), Jurkat, Daudi, Raji and CAP (ATCC Number CRL-1098) cells may also be used for protein production, in order to obtain a native glycosylation pattern for recombinant human proteins.

In one embodiment, the cell line is capable of growing in serum-free medium (*e*.*g*. a chemically-defined medium) and/or in suspension. Such a cell line can easily be obtained by those skilled in the art by adapting the parent cell line to grow in serum-free medium and/or in suspension (*e*.*g*. through single cell cloning, through progressive adaptation and/or through a "starve and save" process).

The cell line of the present invention is a cell line comprising an endogenous dehydroorotate dehydrogenase (DHODH) gene which is partially or fully inactivated and an endogenous glutamine synthetase (GS) gene which is partially or fully inactivated.

By "endogenous DHODH gene" is meant herein a DHODH gene normally present in said particular cell at a particular developmental stage under particular environmental conditions.

The "endogenous DHODH gene" distinguishes from the "exogenous DHODH" defined below, in that said exogenous DHODH is provided by the expression vector defined below, which may be present in the cell line of the invention if said expression vector has been introduced in said cell line.

As will be understood from the skilled person, the endogenous DHODH gene will depend on the cell line. For example, in a CHO cell line, the endogenous DHODH gene is a Chinese hamster DHODH gene; in a human cell line, the endogenous DHODH gene is a human DHODH gene.

Typically, a wild-type Chinese hamster DHODH refers to a sequence comprising or consisting of SEQ ID NO: 2, as well as variants thereof exhibiting DHODH activity. Such variants may for example correspond to variants that occur naturally in hamster species (such as allelic variants or splice variants).

Typically, a wild-type human DHODH refers to a sequence comprising or consisting of SEQ ID NO: 4, as well as variants thereof exhibiting DHODH activity. Such variants may for example correspond to variants that occur naturally in human species (such as allelic variants or splice variants).

By "endogenous GS gene" is meant herein a GS gene normally present in said particular cell at a particular developmental stage under particular environmental conditions.

The "endogenous GS gene" distinguishes from the "exogenous GS" defined below, in that said exogenous GS is provided by the expression vector defined below, which may be present in the cell line of the invention if said expression vector has been introduced in said cell line.

As will be understood from the skilled person, the endogenous GS gene will depend on the cell line. For example, in a CHO cell line, the endogenous GS gene is a Chinese hamster GS gene; in a human cell line, the endogenous GS gene is a human GS gene.

Typically, a wild-type Chinese hamster GS refers to a sequence comprising or consisting of SEQ ID NO: 9, as well as variants thereof exhibiting GS activity. Such variants may for example correspond to variants that occur naturally in hamster species (such as allelic variants or splice variants).

Typically, a wild-type human GS refers to a sequence comprising or consisting of SEQ ID NO: 6, as well as variants thereof exhibiting GS activity. Such variants may for example correspond to variants that occur naturally in human species (such as allelic variants or splice variants).

As used herein, a "gene" includes a DNA region encoding a gene product, as well as all DNA regions which regulate the production of the gene product, whether or not such regulatory sequences are adjacent to coding and/or transcribed sequences. Accordingly, a gene includes promoter sequences, terminators, translational regulatory sequences such as ribosome binding sites and internal ribosome entry sites, enhancers, silencers, insulators, boundary elements, replication origins, matrix attachment sites and locus control regions.

Gene "inactivation" refers to any reduction in gene expression as compared to the corresponding wild-type cell. Gene inactivation may be complete (full inactivation or knock-out) or partial (*e*.*g*. a hypomorph in which a gene exhibits less than normal expression levels or a product of a mutant gene that shows partial reduction in the activity it influences).

In a particular embodiment, one or more or all the alleles of the endogenous DHODH gene are partially or fully inactivated.

In a particular embodiment, said endogenous DHODH gene is fully inactivated.

In a more particular embodiment, one or more or all the alleles of the endogenous DHODH gene are fully inactivated.

In another particular embodiment, one or more or all the alleles of the endogenous GS gene are partially of fully inactivated.

In a particular embodiment, said endogenous GS gene is fully inactivated.

In a more particular embodiment, one or more or all the alleles of the endogenous GS gene are fully inactivated.

In still a particular embodiment, one or more or all the alleles of the endogenous DHODH gene and all the alleles of the endogenous GS gene are fully inactivated.

In a particular embodiment, the endogenous DHODH gene and/or the endogenous GS gene is/are inactivated using the CRISPR-Cas9 method, as described in Aga *et al.* (2015) *BMC Proceedings* **9**(suppl 9):P2.

As well-known from the skilled person, CRISPR-Cas9 system is a prokaryotic adaptive immune response system that uses noncoding RNAs to guide the Cas9 nuclease to induce site-specific DNA cleavage. This DNA damage is repaired by cellular DNA repair mechanisms, either via the non-homologous end joining DNA repair pathway (NHEJ) or the homology-directed repair (HDR) pathway. To create gene disruptions, a single guide RNA (gRNA), consisting of a crRNA sequence that is specific to the DNA target, and a tracrRNA sequence that interacts with the Cas9 protein, binds to a recombinant form of Cas9 protein that has DNA endonuclease activity. The resulting complex will cause target-specific double-stranded DNA cleavage. The cleavage site will be repaired by the nonhomologous end joining (NHEJ) DNA repair pathway, an error-prone process that may result in insertions/deletions (INDELs) that may disrupt gene function.

In a particular embodiment, at least one exon of the DHODH gene is targeted for inactivation, in particular by a gene editing method, such as a CRISPR-Cas9 method. In a more particular embodiment, the part of the DHODH gene encoding the N-terminal part of the DHODH protein is targeted for inactivation, in particular by a gene editing method, such as a CRISPR-Cas9 method. In still another embodiment, the second exon of the DHODH gene is targeted for inactivation, in particular by a gene editing method, such as a CRISPR-Cas9 method.

In one embodiment, a 20-nucleotide sequence of sequence GGATGCAGCCATCATCCTTG (SEQ ID NO: 10) or any sequence compatible with the knocking out of DHODH gene without impairing the CHO survival, is used as the corresponding piece of DNA for generating gRNA, which targets the second exon of the DHODH gene. This gRNA is typically obtained using the oligonucleotides of sequence GGATGCAGCCATCATCCTTGGTTTT (SEQ ID NO: 11) and CAAGGATGATGGCTGCATCCCGGTG (SEQ ID NO: 12), typically cloned at a unique restriction site of a plasmid, such as the *Bael* site of the pCM3561 plasmid (commercialized by Invitrogen), so that the cloned DNA sequence is under the control of the U6 promoter and, once said plasmid is introduced into the cell, is transcribed into a single transcription unit containing a crRNA fused to tracrRNA, the crRNA part being specific of the second exon of the DHODH gene and the tracrRNA part being recognized by the Cas9 enzyme.

In another particular embodiment, at least one exon of the GS gene is targeted for inactivation, in particular by a gene editing method, such as a CRISPR-Cas9 method. In a more particular embodiment, exon 6 the GS gene is targeted for inactivation, in particular by a gene editing method, such as a CRISPR-Cas9 method. In still another embodiment, the vicinity of exon 6 of the GS gene is targeted for inactivation, in particular by a gene editing method, such as a CRISPR-Cas9 method.

In one embodiment, a 18-nucleotide sequence of sequence GAGTATGTGAAGACTTTG (SEQ ID NO: 29) or any sequence compatible with the knocking out of GS gene without impairing the CHO survival, is used as the corresponding piece of DNA for generating gRNA, which targets exon 6 of the GS gene. In another embodiment, a nucleotide sequence of sequence CCATCTTTATTCTCATGGGG (SEQ ID NO: 30) or any sequence compatible with the knocking out of GS gene without impairing the CHO survival, is used as the corresponding piece of DNA for generating gRNA, which targets the vicinity of exon 6 of the GS gene.

In order to identify a cell line inactivated for the DHODH gene and the GS gene, single cells are typically isolated by limiting dilution in well plates, and, after reaching appropriate confluence, for example 90% confluence, the cells are split into at least 2 conditions, such as one in a culture medium supplemented with uridine and glutamine and another in a culture medium devoid of uridine and of uridine. Clones of interest are typically the clones sensitive to the lack of uridine and of glutamine.

Once isolated, these cells of interest can be cultured in a culture medium comprising a pyrimidine base, in particular a culture medium comprising uridine, and further comprising glutamine.

By "pyrimidine base" is meant herein pyrimidine per se and various pyrimidine derivatives having a pyrimidine nucleus as a skeleton. Examples of such pyrimidine bases include uracil nucleic acid-related substances, such as uracil, uridine, uridine phosphates, in particular uridine monophosphate (UMP), uridine diphosphate (UDP) and uridine triphosphate (UTP), deoxyuridine, deoxyuridine phosphates, in particular deoxyuridine monophosphate (dUMP), deoxyuridine diphosphate (dUDP) and deoxyuridine triphosphate (dUTP); cytosine nucleic acid-related substances, such as cytosine, cytidine, cytidine phosphates, in particular cytidine monophosphate (CMP), cytidine diphosphate (CDP), cytidine triphosphate (CTP), deoxycytidine, 2'-deoxycytidine, deoxycytidine phosphates, in particular deoxycytidine monophosphate (dCMP), deoxycytidine diphosphate (dCDP) and deoxycytidine triphosphate (dCTP); thymine, thymidine, thymidine phosphates in particular thymidine monophosphate (TMP) thymidine diphosphate (TDP) and thymidine triphosphate (TTP), deoxythymidine, deoxythymidine phosphates in particular deoxythymidine monophosphate (dTMP), deoxythymidine diphosphate (dTDP) and deoxythymidine triphosphate (dTTP) and orotate.

In a particular embodiment, said pyrimidine base is uridine.

By "uridine" is meant herein the nucleoside of the following formula

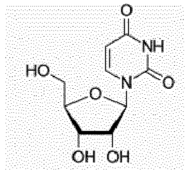

By "glutamine" is meant herein the amino acid of the following formula

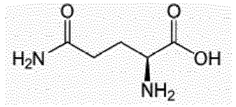

By "culture medium devoid of uridine" is meant any basal culture medium suitable for the growth of a particular cell line, wherein said medium comprises less than 1 mM of uridine, in particular said medium does not comprise any uridine.

By "culture medium devoid of glutamine" is meant any basal culture medium suitable for the growth of a particular cell line, wherein said medium comprises less than 1 mM of glutamine, in particular said medium does not comprise any glutamine.

By "culture medium devoid of uridine and of glutamine" is meant herein any basal culture medium suitable for the growth of a particular cell line, wherein said medium comprises less than 1 mM of uridine and less than 1 mM of glutamine, in particular said medium does not comprise any uridine nor any glutamine.

By "culture medium comprising uridine" is meant any basal culture medium suitable for the growth of a particular cell line, wherein said medium further comprises from 1 mM and 25 mM of uridine, in particular from 5 mM to 10 mM of uridine.

By "culture medium comprising glutamine" is meant any basal culture medium suitable for the growth of a particular cell line, wherein said medium further comprises from 1 mM and 25 mM of glutamine, in particular from 4 mM to 6 mM of glutamine.

By "culture medium comprising uridine and glutamine" is meant any basal culture medium suitable for the growth of a particular cell line, wherein said medium further comprises from 1 mM and 25 mM of uridine, in particular from 5 mM to 10 mM of uridine, and from 1 mM and 25 mM of glutamine, in particular from 4 mM to 6 mM of glutamine.

By "basal culture medium" is meant herein an unsupplemented medium which is suitable for exposure to cells, for example to CHO cells. As will be understood by the skilled person, the basal culture medium to be used will depend of the type of cells used. Examples of basal culture medium include CDCHO medium, OPTiCHO^{™} medium, Fecto CHO^{™} medium, FortiCHO^{™} medium, ExpiCHO^{™} medium, Ex-Cell^{™} medium, ActiPRO^{™} medium, MAM PF77^{™} medium and PowerCHO^{™} medium.

Accordingly, in a particular embodiment, the cell line of the invention is produced by:
(a1) inactivating the endogenous DHODH gene in a cell comprising an endogenous DHODH gene and an endogenous GS gene,
(b1) culturing the cell in a culture medium comprising uridine under conditions suitable for generating a cell line in which the endogenous DHODH gene is partially or fully inactivated,
(c1) recovering said cell wherein the endogenous DHODH gene is partially or fully inactivated,
(d1) inactivating the endogenous GS gene in said cell, and
(e1) culturing said cell in a culture medium comprising glutamine under conditions suitable for generating a cell line in which the endogenous GS gene is partially or fully inactivated.

In an alternative embodiment, the cell line of the invention is produced by:
(a2) providing a cell comprising an endogenous GS gene, and an endogenous DHODH gene which is partially or fully inactivated,
(b2) inactivating the endogenous GS gene is said cell, and
(c2) culturing the cell in a culture medium comprising glutamine under conditions suitable for generating a cell line in which the endogenous GS gene is partially or fully inactivated.

In still an alternative embodiment, the cell line of the invention is produced by:
(a3) providing a cell comprising and endogenous DHODH gene, and an endogenous GS gene which is partially or fully inactivated,
(b3) inactivating the endogenous DHODH gene in said cell, and
(c3) culturing the cell in a culture medium comprising uridine under conditions suitable for generating a cell line in which the endogenous DHODH gene is partially or fully inactivated.

The production of a CHO cell line comprising an endogenous DHODH gene which is fully or partially inactivated by a CRISPR-Cas9 approach, is more deeply exemplified in

### Example 2.

The production of a CHO cell line comprising an endogenous DHODH gene which is fully or partially inactivated and further comprising an endogenous GS gene which is fully or partially inactivated by a CRISPR-Cas9 approach, is exemplified in **Example 3.**

The production of a cell line, such as a CHO cell line, comprising an endogenous DHODH gene which is fully or partially inactivated and an endogenous GS gene which is fully or partially inactivated, can be generated by a variety of other molecular biology techniques known in the art. For example, other gene editing techniques useful for generating a cell line having an endogenous DHODH gene which is fully or partially inactivated and an endogenous GS gene which is fully or partially inactivated include use of zinc finger nucleases (ZFNs) or Transcription Factor-like Effector Nucleases (TALENs). A Cre/Lox method can also be used to knock-out one or more or all alleles of the DHODH gene and of the GS gene.

In a particular embodiment, the cell line of the invention further comprises expression vectors as defined below in the section *"Expression vectors".*

Said expression vectors may be introduced into the cell line by any suitable technique well-known from the skilled person, such as by transfection, in particular by electroporation or chemical transfection, or transduction.

### Exogenous DHODH and exogenous GS

The DHODH encoded by one of the expression vectors used in the present invention (further referred to as "exogenous DHODH") may comprise or consist of a sequence at least 60%, 62%, 65%, 70%, 75%, 80%, 85%, 90%, 91%; 92%; 93%, 94%, 95%, 95.5%, 96%, 96.5%, 97%, 97.5%, 98%, 98.5%, 99%, 99.5% or 100% identical to SEQ ID NO: 2 or SEQ ID NO: 4. It may also comprise or consist of a fragment of at least 100, 150, 200, 250, 300 or 350 consecutive amino acids of SEQ ID NO: 2 or SEQ ID NO: 4, provided the protein retains DHODH activity.

In some embodiments, the exogenous DHODH according to the invention comprises or consists of a sequence at least 60%, 62%, 65%, 70%, 75%, 80%, 85%, 90%, 91%; 92%; 93%, 94%, 95%, 95.5%, 96%, 96.5%, 97%, 97.5%, 98%, 98.5%, 99%, 99.5% or 100% identical both to the sequence of SEQ ID NO: 2 and to the sequence of SEQ ID NO: 4.

In some embodiments, the exogenous DHODH according to the invention is a human DHODH, *i.e.* a DHODH of human origin.

As used herein, the term "human DHODH" refers to a protein of sequence comprising or consisting of SEQ ID NO: 4, as well as variants thereof exhibiting DHODH activity. Such variants may for example correspond to variants that occur naturally in human species (such as allelic variants or splice variants). Alternatively, such variants may correspond to variants obtained by genetic engineering. In one embodiment, such variants only differ from the sequence of SEQ ID NO: 4 by the presence of at most 150, 140, 130, 120, 110, 100, 90, 80, 70, 60, 50, 40, 30, 25, 24, 23, 22, 21, 20, 19, 18,17,16,15,14,13, 12, 11, 10, 9, 8, 7, 6, 5, 4, 3, 2 or 1 amino acid variations as compared to SEQ ID NO: 4 (said variations including substitutions, insertions and deletions).

In a particular embodiment, said human DHODH is a variant comprising a G202A mutation compared to the wild-type sequence, typically a protein comprising or consisting of the amino acid sequence SEQ ID NO: 28.

In some embodiments, the exogenous DHODH is a hamster DHODH, *i.e.* a DHODH of hamster origin. The hamster DHODH may be, for example, Chinese hamster (Cetulus griseus) DHODH.

As used herein, the term "Chinese hamster DHODH" refers to a sequence comprising or consisting of SEQ ID NO: 2, as well as variants thereof exhibiting DHODH activity. Such variants may for example correspond to variants that occur naturally in hamster species (such as allelic variants or splice variants). Alternatively, such variants may correspond to variants obtained by genetic engineering. In one embodiment, such variants only differ from the sequence of SEQ ID NO: 2 by the presence of at most 150, 140, 130, 120, 110, 100, 90, 80, 70, 60, 50, 40, 30, 25, 24, 23, 22, 21, 20, 19, 18, 17, 16, 15, 14, 13, 12, 11, 10, 9, 8, 7, 6, 5, 4, 3, 2 or 1 amino acid variations as compared to SEQ ID NO: 2 (said variations including substitutions, insertions and deletions).

In another embodiment, the variant DHODH will have DHODH activity, optionally the same level of activity as the wild-type protein, or 50%, 60%, 70%, 80%, 90%, 100%, 110%, 120%, 130%, 140% or more of the level of activity as the wild-type protein.

The GS encoded by the other expression vector used in the present invention (further referred to as "exogenous GS") may comprise or consist of a sequence at least 94.5%, 95%, 95.5%, 96%, 96.5%, 97%, 97.5%, 98%, 98.5%, 99%, 99.5% or 100% identical to at least one of SEQ ID NO: 6 or SEQ ID NO: 8. It may also comprise or consist of a fragment of at least 100, 150, 200, 250, 300 or 350 consecutive amino acids of SEQ ID NO: 6 or SEQ ID NO: 8, provided the protein retains its GS activity.

In a specific embodiment, the exogenous GS according to the invention comprises or consists of a sequence at least 94.5 %, 95%, 95.5%, 96%, 96.5%, 97%, 97.5%, 98%, 98.5%, 99%, 99.5% or 100% identical both to the sequence of SEQ ID NO: 6 and to the sequence of SEQ ID NO: 8.

In a specific embodiment, the exogenous GS according to the invention comprises or consists of a sequence at least 97.5%, 98%, 98.5%, 99%, 99.5% or 100% identical to the sequence of SEQ ID NO: 6. As shown in the international application WO2013/186371, such a GS is particularly advantageous for use as a selectable marker in CHO cells, in particular in the E94 CHO cell line.

In a specific embodiment, the exogenous GS according to the invention is a human GS, *i.e.* a GS of human origin. As used herein, the term "human GS" refers to a sequence comprising or consisting of SEQ ID NO: 6, as well as variants thereof exhibiting GS activity. Such variants may for example correspond to variants that occur naturally in human species (such as allelic variants or splice variants). Alternatively, such variants may correspond to variants obtained by genetic engineering. Most preferably, such variants only differ from the sequence of SEQ ID NO: 6 by the presence of at most 22, 20, 15, 10, 9, 8, 7, 6, 5, 4, 3, 2 or 1 amino acid variations as compared to SEQ ID NO: 6 (said variations including substitutions, insertions and deletions).

In another specific embodiment, the exogenous GS according to the invention is a dog GS, *i.e.* a GS of dog origin. As used herein, the term "dog GS" refers to a sequence comprising or consisting of SEQ ID NO: 8, as well as variants thereof exhibiting GS activity. Such variants may for example correspond to variants that occur naturally in dog species (such as allelic variants or splice variants). Alternatively, such variants may correspond to variants obtained by genetic engineering. Most preferably, such variants only differ from the sequence of SEQ ID NO: 8 by the presence of at most 22, 20, 15, 10, 9, 8, 7, 6, 5, 4, 3, 2 or 1 amino acid variations as compared to SEQ ID NO: 8 (said variations including substitutions, insertions and deletions).

In a specific embodiment, the exogenous GS according to the invention comprises at least 1, 2, 3, 4, 5, 6, 10, 15, 16, 20 or 22 of the following amino acids: 12G, 16V, 18M, 19S, 33I, 49S, 80V, 82A, 91K, 116T, 191A, 269Y, 282Q, 35OS, 355L, 356I, 2T, 68L, 98L, 107R, 169R and 2138, wherein the number indicates the position on SEQ ID NO: 6 and SEQ ID NO: 8, and the letter the nature of the amino acid (using the one-letter genetic code). In a more specific embodiment, the exogenous GS according to the invention comprises at least 1, 2, 3, 4, 5 or 6 of the following amino acids: 2T, 68L, 98L, 107R, 169R and 2138. In another more specific embodiment, the exogenous GS according to the invention comprises at least 1, 2, 3, 4, 5, 6, 10, 15 or 16 of the following amino acids: 12G, 16V, 18M, 19S, 33I, 49S, 80V, 82A, 91K, 116T, 191A, 269Y, 282Q, 35OS, 355L and 356I. The above amino acids appear to be specific to the human and/or dog GS, as compared to the CHO GS.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

Sequence identity may be determined over the full length of the variant sequence, the full length of the reference sequence, or both. For example, the percentage of identity may be calculated using a global alignment (i.e. the two sequences are compared over their entire length). Methods for comparing the identity and homology of two or more sequences are well known in the art. The "needle" program, which uses the Needleman-Wunsch global alignment algorithm (Needleman and Wunsch (1970) J. Mol. Biol. 48:443-453) to find the optimum alignment (including gaps) of two sequences when considering their entire length, may for example be used when performing a global alignment. This needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Variants of a reference sequence may comprise mutations such as deletions, insertions and/or substitutions compared to the reference sequence. In case of substitutions, the substitution preferably corresponds to a conservative substitution as indicated in the table below.

| **Conservative substitutions** | **Type of amino acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

### Expression vectors

The expression vectors used in the context of the invention are suitable for the production of recombinant proteins, and comprise a sequence encoding dihydroorotate dehydrogenase (DHODH) or a sequence encoding glutamine synthetase (GS).

The expression vectors are preferably DNA vectors.

One of the expression vectors used in the context of the invention comprises a sequence (i) encoding an exogenous DHODH as defined in section *"Exogenous DHODH and exogenous GS"* above.

In a specific embodiment, the cell line into which the expression vector is to be introduced is a CHO cell line, and the exogenous DHODH is of heterologous origin (*i*.*e*. exogenous DHODH is not a hamster DHODH).

The sequence encoding such an exogenous DHODH may be the naturally occurring nucleotide sequence. Alternatively, the triplet codons of the sequence encoding such a DHODH may be biased for expression in CHO cells. Software and algorithms for biasing sequence in order to obtain an optimal expression are known in the art and include, *e.g.* the algorithm described in Raab et al. (2010) Syst Synth Biol. 4:215-225. This algorithm not only provides the best available codons for expression, but also takes into account the GC content and the absence of non-desired DNA motifs.

For instance, the sequence encoding the exogenous DHODH may comprise or consist of a sequence at least 60%, 62%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% identical to the sequence of SEQ ID NO: 3 (*i.e.* a sequence encoding the human DHODH of SEQ ID NO: 4, which has been designed for optimal expression in CHO cells) and/or to the sequence of SEQ ID NO: 1 (*i.e.* a sequence encoding a hamster DHODH of SEQ ID NO: 2, which has been designed for optimal expression in CHO cells).

In one embodiment, the sequence encoding the exogenous DHODH comprises or consists of a sequence of SEQ ID NO: 1 or SEQ ID NO: 3.

The other expression vector used in the context of the invention comprises a sequence (ii) encoding an exogenous GS as defined in section *"Exogenous DHODH and exogenous GS"* above.

In a specific embodiment, the cell line into which the expression vector is to be introduced is a CHO cell line, and the exogenous GS is of heterologous origin (*i*.*e*. exogenous GS is not a hamster GS).

The sequence encoding such an exogenous GS may be the naturally-occurring nucleotide sequence. Alternatively, the triplet codons of the sequence coding for such a GS may be biased for expression in CHO cells. Software and algorithms for biasing sequence in order to obtain an optimal expression are known in the art and include, *e*.*g*. the algorithm described in Raab et al. (2010) Syst Synth Biol. 4:215-225. This algorithm not only provides the best available codons for expression, but also takes into account the GC content and the absence of non-desired DNA motifs.

For instance, the sequence encoding the exogenous GS may comprise or consist of a sequence at least 80%, 81%, 82%, 83%, 84%, 85%, 86%, 87%, 88%, 89%, 90%, 91%, 92%, 93%, 94%, 95%, 96%, 97%, 98%, 99% or 100% to the sequence of SEQ ID NO: 5 (*i.e.* a sequence encoding the human GS of SEQ ID NO: 6, which has been designed for optimal expression in CHO cells) and/or to the sequence of SEQ ID NO: 7 (*i.e.* a sequence encoding a dog GS of SEQ ID NO: 8, which has been designed for optimal expression in CHO cells).

In one embodiment, the sequence encoding the GS comprises or consists of a sequence of SEQ ID NO: 5 or SEQ ID NO: 7.

In the expression vectors used in the context of the invention, the sequence encoding the exogenous DHODH defined above and/or the sequence encoding the exogenous GS defined above may be placed under the control of any promoter known to those skilled in the art.

For instance, the sequence encoding the exogenous DHODH defined above and/or the sequence encoding the exogenous GS defined above may for example be placed under the control of a promoter suitable for driving respectively expression of DHODH and GS, for instance a Simian vacuolating virus 40 (SV40) promoter (*e*.*g*. the late or the early promoter of SV40), CMV promoter, Elongation Factor 1 promoter, GAPDH promoter, RPL37 promoter, Actin Promoter. An early SV40 promoter is for example described in Benoist and Chambon (1981) Nature 290:304-310 and in Moreau et al. (1981) Nucleic Acids Res. 9:6047-6068. In particular, said SV40 promoter is a full-length promoter. Said SV40 promoter may also have a replication origin containing a 72bp repeat.

In some embodiments, said SV40 promoter is not an SV40 promoter in which positions 128 to 270 have been removed, *i.e.* said SV40 promoter is not the SV40 promoter described in Korean patent No. 10-0267720 and transforming the *E. coli* transformant deposited to the Gene Bank, Institute of Bioengineering, KIST on 17 December 1997 under the Deposition Number: KCTC 8860 P.

In other embodiments, the sequence encoding the exogenous DHODH defined above and/or the sequence encoding the exogenous GS defined above is/are not placed under the control of a SV40 promoter.

Expression vectors that are suitable for the production of recombinant proteins are known to those skilled in the art. Such vectors typically correspond to expression vectors that comprise an origin of replication and at least one expression cassette allowing the cloning and the expression of the recombinant protein for which production is desired. An expression cassette typically comprises a 5' untranslated region (comprising or consisting of a promoter, and optionally an enhancer sequence), one or more restriction sites allowing the cloning of a sequence encoding the recombinant protein, a 3' untranslated region (*e*.*g*. a polyA signal), and optionally one or more introns. The promoter sequence may correspond to any strong promoter well-known to the art, such as *e*.*g*. the human CMV promoter. Optionally, the expression vectors used in the context of the invention comprise a prokaryotic origin of replication (*e*.*g*. a prokaryotic replicon such as ColE1 in *E*. *coli*) and at least a prokaryote-selective marker gene, also known as prokaryotic selectable marker, so that the vectors allows for replication in prokaryotic cells. The cells which replicate the vectors also express the prokaryote-selective marker gene, and therefore can be identified and selected. Prokaryote-selective marker genes are well known to the person skilled in the art. Examples of prokaryote-selective marker genes are for instance nucleic acid sequences encoding a protein conferring antibiotic resistance (*e.g.* a sequence encoding a protein conferring resistance to ampicillin, chloramphenicol, blasticidin or kanamycin).

The recombinant proteins (I) and (II) that can be expressed by the expression vectors of the invention may correspond to any proteins that are of interest to those skilled in the art.

As used herein, the term "protein" is meant to encompass peptides (*i*.*e*. amino acid chains of less than 50 amino acids), polypeptides (*i.e*. amino acid chains of at least 50 amino acids), monomeric proteins (*i*.*e*. proteins consisting of one amino acid chain) and multimeric proteins (*i*.*e*. proteins consisting of two or more amino acid chains, such as *e.g.* monoclonal antibodies).

The expression vectors used in the context of the invention typically comprise a number of expression cassettes that is identical to the number of different amino acid chains that constitute the protein (*e*.*g*. one expression cassette in case of a monomeric protein or homodimeric protein, two in the case of a heterodimeric protein or of a monoclonal antibody, etc.)

Alternatively, the expression vectors used in the context of the invention may comprise only one expression cassette even when production of a heterodimeric protein or of a monoclonal antibody is desired. In such a case, the sequence(s) encoding the other amino acid chain(s) of the protein is (are) present on the other expression vector of the invention.

In particular embodiments, recombinant proteins (I) and (II) are distinct arms of a monoclonal antibody.

Thus, in some embodiments, one of the expression vectors used in the context of invention comprises:
- a sequence encoding exogenous DHODH, as defined above, placed under the control of the early SV40 promoter;
- a first expression cassette, in which the sequence encoding the light chain of a first arm of an antibody is placed under the control of the CMV promoter;
- a second expression cassette, in which the sequence encoding the heavy chain of said first arm of the antibody is placed under the control of the CMV promoter;
- a prokaryotic origin of replication; and
- a selectable marker for use in prokaryotic cells, namely a sequence encoding a protein conferring resistance to ampicillin, placed under the control of its natural promoter.

In that embodiment, the other expression vector used in the context of the invention comprises:
- a sequence encoding exogenous GS, as defined above, placed under the control of the early SV40 promoter;
- a first expression cassette, in which the sequence encoding the light chain of a second arm of the antibody is placed under the control of the CMV promoter;
- a second expression cassette, in which the sequence encoding the heavy chain of said second arm of the antibody is placed under the control of the CMV promoter;
- a prokaryotic origin of replication; and
- a selectable marker for use in prokaryotic cells, namely a sequence encoding a protein conferring resistance to ampicillin, placed under the control of its natural promoter.

Throughout the present specification, the term "recombinant protein" refers to any recombinant protein for which production is desired. It can for example correspond to a therapeutic and/or a prophylactic protein, *i.e.* a protein intended for use as a medicament (including vaccines). In a specific embodiment, the recombinant protein for which production is desired is not a DHODH nor a GS. In another specific embodiment, the recombinant protein for which production is desired is an antibody, for instance a monoclonal antibody. In still another specific embodiment, the recombinant protein for which production is desired is an antigenic protein.

The term "antibody" is used herein in the broadest sense and specifically covers monoclonal antibodies (including full length monoclonal antibodies) of any isotype such as IgG, IgM, IgA, IgD, and IgE, polyclonal antibodies, multispecific antibodies (including bispecific and trispecific antibodies), antibody fragments (such as *e*.*g*. Fv, scFv, ds, Fab, Fab', or F(ab')₂ fragments), single domain antibodies and fragment thereof, and fusion proteins comprising an antibody fragment. An antibody reactive with a specific antigen can be generated by recombinant methods such as selection of libraries of recombinant antibodies in phage or similar vectors, or by immunizing an animal with the antigen or an antigen-encoding nucleic acid.

A "monoclonal antibody", as used herein, is an antibody obtained from a population of substantially homogeneous antibodies, *i.e.* the antibodies forming this population are essentially identical except for possible naturally occurring mutations which might be present in minor amounts. These antibodies are directed against a single epitope (or a single group of epitopes in the case of multispecific monoclonal antibodies) and are therefore highly specific.

A typical monoclonal antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are usually referred to as CDR1, CDR2, and CDR3, numbered sequentially from the N-terminus (see Kabat et al., Sequences of Proteins of Immunological Interest, 5th edition, National Institute of Health, Bethesda, MD, 1991). The more highly conserved portions of the variable regions are called the "framework regions".

The monoclonal antibody may for example be a murine antibody, a chimeric antibody, a humanized antibody, or a fully human antibody.

The monoclonal antibody may be a bispecific or a trispecific antibody.

When the recombinant protein for which production is desired is a monoclonal antibody, the expression vectors according to the invention may comprise a first expression cassette suitable for cloning of an antibody light chain, and a second expression cassette suitable for cloning of the antibody heavy chain.

In a specific embodiment, said first and second expression cassettes each comprise the cytomegalovirus (CMV) promoter, for instance a CMV promoter from a human or a murine CMV. More specifically, said first and second expression cassettes may comprise:
- a CMV immediate early enhancer promoter (e.g. the one having the sequence described in Teschendorf et al. (2002) Anticancer Res. 22:3325-3330); or
- a IE2 promoter/enhancer region from mouse CMV (e.g. the one having the sequence described in Chatellard et al. (2007) Biotechnol Bioeng. 96:106-117); or
- a hCMV-MIE regulatory element (*e*.*g*. the one having the sequence described in WO 89/01036).

The term "antigenic protein" is used herein in the broadest sense and covers any protein capable of generating an immune response, either alone or in combination with an adjuvant. It may be intended for use either in a prophylactic vaccine or in a therapeutic vaccine. In a specific embodiment the antigenic protein is a vaccinal protein, *i.e.* a protein intended for use in a prophylactic vaccine.

The expression vectors may either comprise at least one sequence encoding the recombinant proteins of interest (*e*.*g*. one sequence encoding a monomeric protein, one sequence encoding an antibody chain, or two sequences, encoding an antibody light chain and an antibody heavy chain, respectively), or they may be empty *(i.e.* devoid of such a sequence encoding the recombinant protein of interest).

### Expression system, kits, methods and uses

The present invention provides an expression system comprising:
(A) the cell line as defined in the section *"Cell line"* above comprising an endogenous DHODH gene which is partially or fully inactivated and an endogenous GS gene which is partially or fully inactivated, as defined in the section *"Cell line"* above,
(B) an expression vector comprising a nucleotide sequence (i) encoding a mammalian DHODH and at least one expression cassette for expressing a recombinant protein (I), wherein said DHODH comprises a sequence at least 60% identical to the sequence SEQ ID NO: 2 or to the sequence SEQ ID NO: 4 as defined in the section *"Expression vectors"* above, and
(C) an expression vector comprising a nucleotide sequence (ii) encoding a mammalian GS ad at least one expression cassette for expressing a recombinant protein (II), wherein the GS comprises a sequence at least 94.5% identical to the sequence SEQ ID NO: 6 or the sequence SEQ ID NO: 8 as defined in the section *"Expression vectors"* above.

The invention provides a kit comprising (i) the cell line according to the invention comprising the expression vectors as defined in the section *"Expression vectors"* above, or the expression system according to the invention, and (ii) a culture medium devoid of uridine and of glutamine, as defined above.

The kit may comprise an exogenous DHODH-encoding expression vector and/or an exogenous GS-encoding expression vector (in the expression system) as described above. In such a kit, the vectors are preferably empty, since this allows the cloning of the proteins of interest for those skilled in the art. In addition, the expression vectors are preferably isolated from the cell line in such a kit.

The kit further comprises a culture medium devoid of uridine and of glutamine, as defined in the section *"Cell line"* above.

The kit may further comprise media suitable for cultivation of the cell line, media suitable for transfection of the vectors into the cell line, a packaging material and/or instructions for use of the expression system.

In a particular embodiment, the kit is devoid of DHODH inhibitor and/or of GS inhibitor.

Examples of DHODH inhibitors include bicinchoninic acid, brequinar (6-fluoro-2-(2'-fluoro-1,1'-biphenyl-4-yl)-3-methyl-4-quinoline carboxylic acid), naphthoquinone derivatives such as dichloroally lawsone, isoxazole derivatives such as leflunomide (5-methyl-*N*-[4-(trifluoromethyl) phenyl]-isoxazole-4-carboxamide) and its active metabolite teriflunomide ((2Z)-2-cyano-3-hydroxy-N-[4-(trifluoromethyl)phenyl]but-2-enamide), quinolone carboxylic acids, naphthoquinones, isoxazoles, phenoxyquinolines, redoxal and derivatives, lawsone, lapachol, atovaquone and (8-chloro-4-(2-chloro-4-fluoro-phenoxy)quinoline). An inhibitor of DHODH may be able to inhibit DHODH activity by at least 20, 30, 40, 50, 60, 70, 80, 90, 95, 99 or 100%.

In a particular embodiment, the kit is devoid of teriflunomide.

Examples of GS inhibitors include methionine sulfoximine, methionine sulfone, phosphinothricin, tabtoxinine-β-lactam, α-methyl methionine sulfoximine, α-ethyl methionine sulfoximine, ethionine sulfoximine, α-methyl ethionine sulfoximine, prothionine sulfoximine, α-methyl prothionine sulfoximine, γ-hydroxy phosphinothricin, γ-acetoxy phosphinothricin, α-methyl phosphinothricin, α-ethyl phosphinothricin, cyclohexane phosphinothricin, cyclopentane phosphinothricin, tetrahydrofuran phosphinothricin, s-phosphonomethyl homocysteine, s-phosphonomethyl homocysteine sulfoxide, s-phosphonomethyl homocysteine sulfone, 4-(phosphonoacetyl)-L-α-aminobutyrate, threo-4-hydroxy-D-glutamic acid, threo-4-fluoro-D,L-glutamic acid, erythro-4-fluoro-D,L-glutamic acid, 2-amino-4-[(phosphonomethyl)hydroxyphosphinyl] butanoic acid, alanosine, 2-amino-4-phosphono butanoic acid, 2-amino-2-methyl-4-phosphono butanoic acid, 4-amino-4-phosphono butanoic acid, 4-amino-4-(hydroxymethylphosphinyl) butanoic acid, 4-amino-4-methyl-4-phosphono butanoic acid, 4-amino-4-(hydroxymethylphosphinyl)-4-methyl butanoic acid, 4-amino-4-phosphono butanamide, 2-amido-4-phosphono butanoic acid, 2-methoxycarbonyl-4-phosphono butanoic acid, methyl 4-amino-4-phosphono butanoate, oxetin, IF7 and IF17 polypeptides, (3-amino 3-carboxypropyl)-(phosphonomethyl) phosphinic acid, N-hydroxy-L-2,4-diaminobutyrate, 3-amino-3-carboxypropane-sulfonamide and 5-hydroxylysine. An inhibitor of GS may be able to inhibit GS activity by at least 20, 30, 40, 50, 60, 70, 80, 90, 95, 99 or 100%.

In a particular embodiment, the kit is devoid of methionine sulfoximine.

The invention further provides the use of the cell line according to the invention comprising the expression vectors as defined in the section *"Expression vectors"* above, the expression system according to the invention, or the kit according to the invention, for producing recombinant proteins *in vitro.*

In a particular embodiment, said cell line, expression system or kit is used in combination with a culture medium devoid of uridine and of glutamine as defined above, more particularly in the absence of a DHODH inhibitor and/or of a GS inhibitor.

The invention further provides the use of the expression system according to the invention, of the cell line according to the invention comprising the expression vectors as defined in the section *"Expression vectors"* above, or of the kit according to the invention, for isolating a clone cell which produces high levels of recombinant proteins ("high producing clones") *in vitro,* in particular in the absence of a DHODH inhibitor and/or of a GS inhibitor.

In the context of the invention, the term "high level of a recombinant protein" is intended to mean that in the culture medium the concentration of recombinant protein is of at least 0.05 g/l, preferably at least 0.1 g/l, still preferably at least 0.2 g/l, more preferably between 0.3 and 1 g/l. The concentration of recombinant protein can be determined by methods which are well known to the person skilled in the art, including in particular Enzyme-linked immunosorbent assay (ELISA), Western blot, a caliper technology and a range of concentration of the purified protein corresponding to the recombinant protein.

The invention further provides an *in vitro* method of producing a recombinant protein comprising the steps of:
A)
   a1) providing a cell line according to the invention comprising the expression vectors as defined in the section *"Expression vectors"* above;
      or
   a2) providing a cell line according to the invention, and
   a2') introducing expression vectors as defined in the section *"Expression vectors"* above into the cell line provided in step a2);
      or
   a3) providing a cell line comprising an endogenous DHODH gene and an endogenous GS gene,
   a3') partially or fully inactivating the endogenous DHODH gene and the endogenous GS gene in the cell line provided in step a3), and
   a3") introducing expression vectors as defined in the section *"Expression vectors"* above into the cell line comprising a partially or fully inactivated endogenous DHODH gene and a partially or fully inactivated GS gene obtained in step a3');
      or
   a4) providing a cell comprising an endogenous GS gene, and an endogenous DHODH gene which is partially or fully inactivated,
   a4') partially or fully inactivating the endogenous GS gene in the cell line provided in step a4), and
   a4") introducing expression vectors as defined in the section *"Expression vectors"* above into the cell line comprising a partially or fully inactivated endogenous DHODH gene and a partially or fully inactivated GS gene obtained in step a4');
      or
   a5) providing a cell comprising an endogenous DHODH gene, and an endogenous GS gene which is partially or fully inactivated,
   a5') partially or fully inactivating the endogenous DHODH gene in the cell line provided in step a5), and
   a5") introducing expression vectors as defined in the section *"Expression vectors"* above into the cell line comprising a partially or fully inactivated endogenous DHODH gene and a partially or fully inactivated GS gene obtained in step a5');
B) culturing said cell line under conditions suitable for production of the recombinant proteins; and
C) isolating and/or purifying said recombinant proteins.

In a particular embodiment, step B) of the above method is conducted in a culture medium devoid of uridine and of glutamine, more particularly also devoid of DHODH inhibitor and/or of GS inhibitor, and in particular comprises a sub-step consisting in selecting the transfected cells which grow despite the absence of uridine and of glutamine, in particular further in the absence of DHODH inhibitor and/or of GS inhibitor.

The invention further provides an *in vitro* method of isolating a clone cell which produces high levels of recombinant protein, said method comprising or consisting of the following steps:
A)
   a1) providing a cell line according to the invention comprising the expression vectors as defined in the section *"Expression vectors"* above;
      or
   a2) providing a cell line according to the invention, and
   a2') introducing expression vectors as defined in the section *"Expression vectors"* above into the cell line provided in step a2);
      or
   a3) providing a cell line comprising an endogenous DHODH gene and an endogenous GS gene,
   a3') partially or fully inactivating the endogenous DHODH gene and the endogenous GS gene in the cell line provided in step a3), and
   a3") introducing expression vectors as defined in the section *"Expression vectors"* above into the cell line comprising a partially or fully inactivated endogenous DHODH gene and a partially or fully inactivated GS gene obtained in step a3');
      or
   a4) providing a cell comprising an endogenous GS gene, and an endogenous DHODH gene which is partially or fully inactivated,
   a4') partially or fully inactivating the endogenous GS gene in the cell line provided in step a4), and
   a4") introducing expression vectors as defined in the section *"Expression vectors"* above into the cell line comprising a partially or fully inactivated endogenous DHODH gene and a partially or fully inactivated GS gene obtained in step a4');
      or
   a5) providing a cell comprising an endogenous DHODH gene, and an endogenous GS gene which is partially or fully inactivated,
   a5') partially or fully inactivating the endogenous DHODH gene in the cell line provided in step a5), and
   a5") introducing expression vectors as defined in the section *"Expression vectors"* above into the cell line comprising a partially or fully inactivated endogenous DHODH gene and a partially or fully inactivated GS gene obtained in step a5');
B) culturing said cell line under conditions suitable for production of the recombinant protein; and
C) isolating a clone which produces high levels of a recombinant protein.

In a particular embodiment, step B) of the above method is conducted in a culture medium devoid of uridine and of glutamine, more particularly also devoid of DHODH inhibitor and/or of GS inhibitor, and in particular comprises a sub-step consisting in selecting the transfected cells which grow despite the absence of uridine and of glutamine, in particular further in the absence of DHODH inhibitor and/or of GS inhibitor.

Said expression vectors can be introduced into said cell line, in steps a2'), a3"), a4") or a5") by any technique well-known from the skilled person, such as by transfection, in particular by electroporation or chemical transfection, or transduction.

Conditions suitable for production of recombinant proteins are well-known to those skilled in the art. The protocols described in the Examples may for instance be used.

In a specific embodiment, the culture medium used in step B) comprises decreasing concentrations of uridine and decreasing concentrations of glutamine. This allows selecting clones in which the vector-derived exogenous DHODH gene and the vector-derived exogenous GS gene (and thus the sequences encoding the recombinant proteins) have been amplified.

The above methods may further comprise the step of formulating the recombinant proteins into a pharmaceutical composition.

Throughout the specification, terms such as "comprises", "comprised" and "comprising" have the meaning attributed to them in most patent jurisdictions, preferably in the jurisdiction in question; *e*.*g*. they can mean "includes", "included", "including", etc. Terms such as "consisting of", "consisting essentially of" and "consists essentially of" have the meaning ascribed to them in most patent jurisdictions, preferably in the jurisdiction in question; e.g. they imply the exclusion of all, most or all but a negligible amount of other elements, they allow for elements not explicitly recited, but exclude elements that are found in the prior art or that affect a basic or novel characteristic of the invention.

Several documents are cited throughout the text of this specification. Each of the documents cited herein (including any journal article or abstract, published or unpublished patent application, issued patent, manufacturer's specifications, instructions, etc.) are hereby incorporated by reference. However, there is no admission that any document cited herein is indeed prior art in respect of the present invention.

The invention will further be described by reference to the following drawings and examples, which are illustrative only, and are not intended to limit the present invention.

The invention is defined by the claims, which should be interpreted with the help of the description and the drawings.

### Brief description of the sequences

| **SEQ ID NO:** | **Description** |
|---|---|
| 1 | cDNA sequence encoding DHODH of Chinese hamster (*Cricetulus griseus)* origin |
| 2 | amino acid sequence of DHODH of Chinese hamster origin |
| 3 | cDNA sequence encoding DHODH of human origin |
| 4 | amino acid sequence of DHODH of human origin |
| 5 | cDNA sequence encoding GS of human origin |
| 6 | amino acid sequence of GS of human origin |
| 7 | cDNA sequence encoding GS of dog (*Canis lupus*) origin |
| 8 | amino acid sequence of GS of dog origin |
| 9 | amino acid sequence of GS of Chinese hamster origin |
| 10 | corresponding piece of DNA for generating DHODH gRNA |
| 11 | oligonucleotide used for obtaining DHODH gRNA (Sequence1') |
| 12 | oligonucleotide used for obtaining DHODH gRNA (Sequence1') |
| 13 | oligonucleotide used for obtaining DHODH gRNA (Sequence2') |
| 14 | oligonucleotide used for obtaining DHODH gRNA (Sequence2') |
| 15 | oligonucleotide used for obtaining DHODH gRNA (Sequence3') |
| 16 | oligonucleotide used for obtaining DHODH gRNA (Sequence3') |
| 17 | oligonucleotide used for obtaining DHODH gRNA (Sequence4') |
| 18 | oligonucleotide used for obtaining DHODH gRNA (Sequence4') |
| 19 | oligonucleotide used for obtaining DHODH gRNA (Sequence5') |
| 20 | oligonucleotide used for obtaining DHODH gRNA (Sequence5') |
| 21 | oligonucleotide used for obtaining DHODH gRNA (Sequence6') |
| 22 | oligonucleotide used for obtaining DHODH gRNA (Sequence6') |
| 23 | oligonucleotide used for obtaining DHODH gRNA (Sequence7') |
| 24 | oligonucleotide used for obtaining DHODH gRNA (Sequence7') |
| 25 | oligonucleotide used for obtaining DHODH gRNA (Sequence8') |
| 26 | oligonucleotide used for obtaining DHODH gRNA (Sequence8') |
| 27 | CHO DHODH gene sequence |
| 28 | amino acid sequence of human DHODH G202A |
| 29 | DNA sequence targeted by GS-1 gRNA |
| 30 | DNA sequence targeted by GS-2 gRNA |
| 31 | CHO GS gene sequence |
| 32 | GS1_F primer |
| 33 | GS1_R primer |
| 34 | cDNA sequence encoding of GS Chinese hamster origin |
| 35 | GS F primer of example 5 |
| 36 | GS R primer of example 5 |

### Examples

### Example 1: Obtaining the CHO 9E4 cell line

This example describes the obtaining of the CHO 9E4 cell line from the CHO-K1 cell line commercially available from the ATCC under the Number ATCC CCL-61.

### 1. The CHO-K1 cell line

A vial of CHO-K1 cells (ATCC CCL-61) frozen in the presence of calf serum in 1969 was obtained from the ATCC.

### 2. Thawing of the vial in Ex-Cell^{™} 302 medium and preparation of the CHO-LG-APF bank

The CHO-K1 vial was thawed directly in Ex-Cell^{™} 302 medium (SAFC) supplemented with 4 mM glutamine and amplified on static support, then in spinner. The resulting CHO-LG-APF bank was frozen in Ex-Cell^{™} 302 medium after 12 passages and 17.3 generations.

### 3. Thawing of the CHO-LG-APF bank in Ex-Cell^{™} 302 medium and preparation of the ABC-024 P22 bank

The CHO-LG-APF vial was thawed in Ex-Cell^{™} 302 medium and amplified. The resulting ABC-024 P22 bank was thawed after 18.5 generations.

### 4. Adapting the CMV07-024 bank to CDCHO Fusion medium and preparation of the ABC-003 bank

The CMV07-024 bank was thawed and directly adapted to Ex-cell^{™} CDCHO Fusion medium (SAFC) supplemented with 4 mM Glutamine and adapted in shaker over 12.5 generations until freezing the ABC-003 bank in Ex-cell^{™} CDCHO Fusion medium.

### 5. Thawing the ABC-003 bank in CDCHO Fusion medium and preparation of the ABC-053 bank in CDCHO Fusion medium

The ABC-003 vial was thawed in CDCHO Fusion medium, and, after a dilution, the ABC-53 bank was frozen after 4.2 generations.

### 6. Thawing the ABC-053 bank in CDCHO Fusion medium, selection, cloning and preparation of the P15A11 bank in CDCHO Fusion medium

The ABC-053 bank was thawed in Ex-cell^{™} CDCHO Fusion medium (SAFC) supplemented with 4 mM glutamine. After amplification of the culture, it was cloned by limiting dilution in plates, and then amplified in CDCHO Fusion medium. The bank of the clone P15A11 resulting from this cloning was frozen. This cloning and amplification corresponds to about 94 generations.

### 7. Thawing the P15A11 bank in CDCHO Fusion medium, adapting the bank by direct passage in CDCHO and preparing the CHOSP10-002 bank in CDCHO medium

The P15A11 bank was thawed in Ex-cell^{™} CDCHO Fusion medium (SAFC) supplemented with 4 mM glutamine, and after 2 passages in CDCHO Fusion medium, the cells were diluted in CDCHO medium. After 3 passages in CDCHO medium, the CHOSP10-002 bank was frozen after a total of 15.9 generations.

### 8. Thawing the CHOSP10-002 bank in CDCHO medium, amplification, elimination of masses by centrifugation and selection by subculture without masses in 96-well plates, amplification in 6-well plates and in shaker to prepare the CHOSP10-012 bank in CDCHO medium

The CHOSP10-002 bank was thawed in CDCHO medium (Invitrogen) supplemented with 6 mM glutamine, then amplified. The culture was centrifuged in order to eliminate cellular masses and continue the culture with only cells isolated from the supernatant. At this stage, from thawing, 11.3 generations were generated.

This culture was split in 96-well plates at 10 cells per well. The wells with cells which multiply isolately in suspension were amplified in 6-well plates, then in shaker. There were 23.2 additional generations until the CHOSP10-012 bank was frozen.

### 9. Thawing the CHOSP10-012 bank, amplification and preparation of the CHOSP11-008 bank (9E4 bank)

The CHOSP10-012 bank was thawed in CDCHO medium (Invitrogen) supplemented with 6 mM glutamine, then amplified from the Erlenmeyer stage to the 17 L bioreactor.

The 9E4 bank was frozen after a total of 10 generations.

### Example 2: Production of a CHO cell line wherein the DHODH gene is invalidated

### A - Design and Construction of CRISPR CAS9 guide RNA (gRNA)

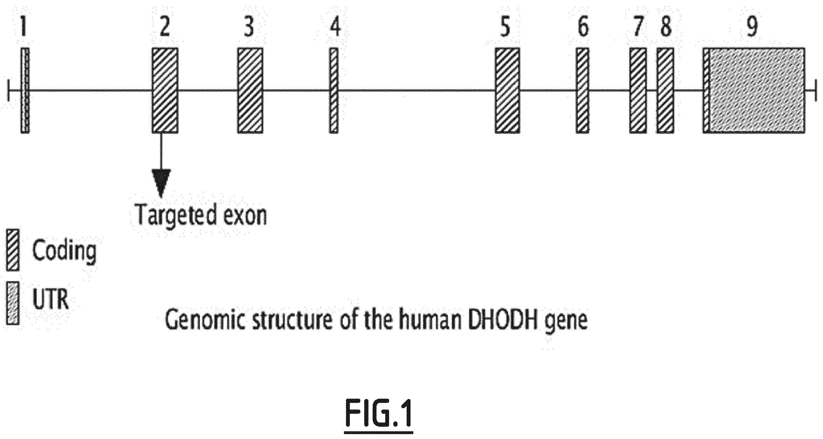

To invalidate the DHDOH gene in CHO cells, the inventors started by recovering the hamster DHODH gene sequence and using the publically available Tefor software for designing different guided RNA (gRNA) for transfection with CRISPR-Cas9 in the CHO genome. The whole CHO DHODH sequence, with introns and exons, is shown in **Figure 1****.**

The software determined 8 sequences that could target the DHODH gene:
Sequence1'
   GGATGCAGCCATCATCCTTGGTTTT (SEQ ID NO: 11)
   CAAGGATGATGGCTGCATCCCGGTG (SEQ ID NO: 12)
Sequence2'
   GATGCAGCCATCATCCTTGGGTTTT (SEQ ID NO: 13)
   CCAAGGATGATGGCTGCATCCGGTG (SEQ ID NO: 14)
Sequence3'
   GCAGCCATCATCCTTGGGGGGTTTT (SEQ ID NO: 15)
   CCCCCAAGGATGATGGCTGCCGGTG (SEQ ID NO: 16)
Sequence4'
   GCCATCATCCTTGGGGGAGGGTTTT (SEQ ID NO: 17)
   CCCCCCAAGGATGATGGCCGGTG (SEQ ID NO: 18)
Sequence5'
   GCTATTCGCTTCACGTCCCTGTTTT (SEQ ID NO: 19)
   AGGGACGTGAAGCGAATAGCCGGTG (SEQ ID NO: 20)
Sequence6'
   GCCTCTACAAACTGGGCTTTGTTTT (SEQ ID NO: 21)
   AAAGCCCAGTTTGTAGAGGCCGGTG (SEQ ID NO: 22)
Sequence7'
   GGCTTTGGGTTTGTCGAGGTGTTTT (SEQ ID NO: 23)
   ACCTCGACAAACCCAAAGCCCGGTG (SEQ ID NO: 24)
Sequence8'
   GCTGGTCTGAGGAGCCTACAGTTTT (SEQ ID NO: 25)
   TGTAGGCTCCTCAGACCAGCCGGTG (SEQ ID NO: 26)

Although the 8 sequences were tested and cloned, among the 8 sequences, only four cloned sequences were transfected and only one was successful for generating a knock-out of the DHODH gene. The following 20 nucleotide sequence was used GGATGCAGCCATCATCCTTG (SEQ ID NO: 10) as the corresponding piece of DNA for generating the gRNA. It is targeting the second exon of the DHODH gene. To obtain the transcription of the proper gRNA, two oligonucleotides GGATGCAGCCATCATCCTTGGTTTT (oligo1', SEQ ID NO: 11) and CAAGGATGATGGCTGCATCCCGGTG (oligo2', SEQ ID NO: 12) were synthetically made, annealed and cloned at the unique *Bael* site of pCM3561 (commercialized by Invitrogen).

The cloned DNA sequence was thereby under the control of the U6 promoter and once the DNA was transfected in CHO cells, it was transcribed into a single transcription unit containing a crRNA fused to tracrRNA. The crRNA part was specific to the second exon of DHODH gene while the tracrRNA was recognized by the Cas9 enzyme itself.

### B-Preparation of the material for CRISPR-Cas9 gene editing

CHO 9E4 cells were isolated and selected from the CHO K-1 cells purchased from ATCC, as disclosed in Example 1, and were grown and maintained as suspension cultures in CDCHO serum-free and chemically-defined medium optimized for the growth of Chinese Hamster Ovary (CHO) cells supplemented with 6 mM L-glutamine at 37°C in an incubator with 8% CO₂ and 80% humidity.

10 µg of sgRNA expressing vector (pCM3561) were digested with 1 µL of *Bael* enzyme at 5 units/µl supplement with 20 µM S-adenosylmethionine (SAM) at 25°C for 1 hour, then the digested plasmid was separated by electrophoresis using 1% agarose gel. The resulting sgRNA cloning vector was then recovered by gel extraction kit (Qiagen Kit).

sgRNA cloning vector and annealed guide oligonucleotides were ligated using the T4 DNA ligase enzyme (Biolabs) and incubated for 10 min at room temperature.

5 µL of ligation products were added to 50 µL of *E. coli* DH5a competent cells (invitrogen).

Cells and DNA were incubated 30 min on ice, and then heat shocked at 42°C for 45 s. After adding 500 mL S.O.C medium, the 1-hour incubation at 37°C (at 800 rpm) gave the bacteria time to generate the antibiotic resistance proteins encoded on the plasmid backbone. After the incubation, each tube was spread on one coated LB supplemented with 100 µg/mL ampicillin. The dishes were incubated overnight at 37°C. Negative controls (with water instead of insert DNA) were used to evaluate the success of the transformation.

For the amplification step, two colonies were chosen per construction and seeded in 2 mL of LB medium supplemented with 100 µg/ml of ampicillin in tube placed in the incubator overnight (at 37°C, 700 rpm). The overnight-incubated culture was harvested by centrifugation. The QlAprep Miniprep Kit^{™} (QIAGEN) was used to recover the amplified DNA (elution in EB buffer). The sequence of the guide oligonucleotides of interest were then checked by Sanger sequencing (sense and antisense sequencing, GATC Company). After verification by alignment on Vector NTI software (Thermofisher Scientific), the corresponding colonies were used to seed 200 mL of LB medium supplemented with 100 µg/ml of ampicillin. After 24 hours incubation, bacteria were harvested by centrifuging at 6000 g for 15 min at 4°C. The EndoFree Plasmid Maxi Kit^{™} (QIAGEN) was used to prepare a MaxiPrep. DNA was precipitated by adding room temperature isopropanol. After a 1 h-centrifugation (at 4°C, 8000 rpm), the DNA pellet was washed by endotoxin-free room temperature 70% ethanol. After a short new centrifugation, the pellet was air-dried during 1 h and re-dissolved in a suitable volume of endotoxin-free sterile water to get a DNA concentration at 5 mg/mL. A nanodrop device was used to measure the DNA concentration.

Four different plasmids were prepared, namely the pBH6840 plasmid (KO DHODH Sequence1'), the pBH6841 plasmid (KO DHODH Sequence2'), the pBH6842 plasmid (KO DHODH Sequence5') and the pBH6843 plasmid (KO DHODH Sequence7'). The targets in the CHO DHODH gene are shown on sequence SEQ ID NO: 27.

### C-CRISPR-Cas9 gene editing

The transfections were made by electroporation using MaxCyte STX and its CHO defined protocol. They were made in OC-100 (20 million cells per transfection) processing assemblies.

The day before transfection, cells were seeded at 1.5×10⁶ cells/mL in CDCHO medium complemented with 6 mM L-Glutamine.

The day of the transfection, cells were numbered with the ViCell apparatus (Beckman & Coulter). The needed number of cells was centrifuged at 250 g for 10 min and the supernatant was thrown away.

For each transfection condition, 20×10⁶ cells were centrifuged 10 min at 250 g. The pellet was resuspended with 70 µL Maxcyte buffer. 30 µg of DNA was added and the mix (cells, buffer and DNA) was transferred into a 100 µL Maxcyte electroporation cassette. The processing assembly used was the OC-100 specific to 100 µL cassette, and the optimized program for CHO was selected.

The following transfections were made.

| | | |
|---|---|---|
| T1 | pBH6840 | KO DHODH Sequence1' |
| T2 | pBH6841 | KO DHODH Sequence4' |
| T3 | pBH6842 | KO DHODH Sequence5' |
| T4 | pBH6843 | KO DHODH Sequence7' |
| T5 | W/O ADN | H2O |

After electroporation, cells were transferred in 25 mL working Erlenmeyer flasks. They were put in a 37°C, 5% CO₂ static incubator for 45 min. 25 mL of CDCHO medium complemented with 6 mM L-Glutamine were then added to resuspend the cells and the Erlenmeyer flasks were put in 37°C, 5% CO₂, 70% humidity, 110 rpm shakers.

The day after electroporation, single cell per well were seeded by limiting dilution from the CHO9E4 transfected pools described above. After about 20 days, once the cells were approximately 90% confluent and appeared healthy when examined under the microscope, the cells were split into 2 new 96 well plates, with or without uridine.

Several clones were selected for their sensitivity to the lack of uridine. These clones were adapted for growth in CDCHO medium complemented with 6 mM of glutamine and 5 mM of uridine.

To confirm that the gene editing was successful, genomic DNA was extracted from the CRISPR-Cas9 clone cells using the Qiagen DNeasy kit^{™} (Qiagen). The target locus was amplified by PCR using the appropriate primers for the region of the DHODH locus targeted by CRISPR-Cas9, and the PCR products were sequenced by NGS using PCR fragments covering the potential deleted regions.

The clones KO2 and KO19 knockout for DHODH were produced thereby.

### Example 3: Production of a CHO cell line wherein the DHODH gene and the GS gene are invalidated

### A - Design and Construction of CRISPR-Cas9 guide RNA (gRNA)

To invalidate the GS gene in CHO KO DHODH KO2 and KO19 cell lines obtained in Example 2, the inventors started by recovering the Chinese hamster GS gene sequence and using the publicly available Tefor software for designing two different guided RNA (gRNA) for transfection with CRISPR-Cas9 protein in the CHO genome.

The software determined 2 optimal sequences that could target the GS gene. Two targeted sequences were chosen, one targeting exon 6 and the other targeting the vicinity of exon6 of the CHO GS gene.

The GS-1 gRNA sequence targets gagtatgtgaagactttg (SEQ ID NO: 29) followed by PAM sequence (ngg) within the CHO GS genome sequence.

The GS-2 gRNA sequence targets ccatctttattctcatgggg (SEQ ID NO: 30) followed by PAM sequence (ngg) within the CHO GS genome sequence.

The targets within the CHO GS gene are shown on sequence SEQ ID NO: 31.

### B-Preparation of the material for CRISPR-Cas9 gene editing

The two KO2 and KO19 knockout DHODH clones were diluted 24 h before transfection in pre-warmed CD CHO medium supplemented with 6 mM glutamine and 5 mM Uridine to achieve a final cell density of 1.5×10⁶ cells/mL in the desired final volume. The flasks were incubated in a 37°C incubator containing a humidified atmosphere of 8% CO₂ in air on a 5 mm orbital shaker platform rotating at 115 rpm.

Under classical conditions of a molecular biology laboratory, crRNA::tracrRNA complexes were prepared as follow respectively for each crRNA - (one for GS-1 gRNA and one for GS-2 gRNA); The two specific crRNA , the constant tracRNA and the purified spCAS9 protein were provided by Integrated DNA Technologies (IDT, Leuven, Belgium)

### cr:tracrRNA mixes

| **Component** | **Amount x1** |
|---|---|
| **crRNA** | 5 µl (500 pmoles) |
| **tracrRNA** | 5 µl (500 pmoles) |
| **Total** | 10 µl |

1. The mixes were Incubated at 95°C for 5 min
   a. Setting of thermocycler as follows:
      i. 95°C 5 min
      ii. 20°C hold (then placed on benchtop)
2. The complex was cooled at room temperature and the RNP was prepared as follows:

### RNP complexes

| **Component** | **Amount x1 (µl)** |
|---|---|
| **cr:RNA/tracrRNA mix** | 10 |
| **SpCas9** | 7 (70µg) |
| **Total** | 17 |

All the components were mixed and incubated at room temperature for 20 min, and during incubation, cells were counted. A sufficient number of cells were harvested counting 1 million cells per transfection experiment. The cultivated cells were centrifuged for 10 min at 250 g, at room temperature and the supernatant was carefully removed without disturbing the cell pellet.

### C-CRISPR-Cas9 gene editing

### Transfection mixes

| **Component** | **Amount x1 (µl)** |
|---|---|
| **RPN complex** | 17 |
| **MaxCyte buffer** | 73 |
| **Total** | 90 |

For each transfection condition, the 1×10⁶ cells pellet was resuspended with the 90 µl transfection mix (RNP complex and MaxCyte buffer containing the crRNA::tracrRNA complexes) and transferred into a 100 µl Maxcyte electroporation cassette.

The processing assembly used was the OC-100 specific to 100 µl cassette, and the optimized program for CHO was selected. The cells were electroporated using this specific Maxcyte designed program.

For the recovery phase, transfected cells were immediately transferred in a 125 ml flask at 37°C, 40 min without agitation. 25 ml pre-warmed CD CHO complemented with 6 mM glutamine and 5 mM uridine was added and the transfected cultures were maintained at 37°C in an incubator with 8% CO₂ and 80% humidity.

At one day post-transfection, single cell per well were seeded in a 96 well plate corresponding to 0.5 cell per well by limiting dilution of the KO2 and KO19 DHODH clones transfected pools described above. The 96 well plates containing the growing potential clones were maintained at 37°C in an incubator with 8% CO₂ and 80% humidity.

After 15 to 25 days of growth, cell occupied well were observed and once the cells were approximately 90% confluent and appeared healthy when examined under the microscope, the growing cells were split into two new 96 well plates, with or without glutamine to verify their glutamine auxotrophy.

Ninety six clones were selected for their sensitivity to the lack of glutamine. These clones were adapted for growth in CD CHO complemented with 6 mM glutamine and 5 mM uridine.

To confirm that the gene editing was successful, genomic DNA was extracted from the CRISPR-Cas9 clone cells and the disruption of the two GS gene alleles was verified by targeted NGS sequencing.

Namely, genomic DNA (gDNA) was prepared using a Pure Link^{™} genomics DNA mini kit (Invitrogen Catalog Number K1820-01) using 1 to 5 × 10⁶ cloned CHO cells.

More specifically, 5 × 10⁶ cells were centrifuged for 10 min at 250 g, at room temperature and the growth medium was carefully removed. The pellet was resuspended in 200 µl PBS. 20 µl of Proteinase K / RNase, and 200 µl of Lysis / Binding Buffer were added to the sample and incubated at 55°C for 10 min. The sample tube was centrifuged for 1 min at 10,000 g. 200 µl of 95-100% ethanol was added to the lysate then it was vortexed. A spin column was added to a collection tube and the prepared lysate was added to the spin column. The spin column and collection tube combination were centrifuged for 1 min at 10,000 g at room temperature then the flow-through was discarded. Next, 500 µl of Wash Buffer 1 was added, the spin column and collection tube combination were centrifuged for 1 min at 10,000 g at room temperature and the flow-through was discarded. Next, 500 µl of Wash Buffer 2 was added, the spin column and collection tube combination were centrifuged for 3 min at 10,000 g at room temperature and the flow-through was discarded. The spin column was added to a new microfuge tube and 100 µl of Elution Buffer was added to the spin column. The sample incubated at room temperature for 1 min then centrifuged for 1 min at maximum speed at room temperature. The column was removed and discarded since the extracted DNA was collected in the microfuge tube.

Starting from 50 ng of genomic DNA, the region of interest corresponding to the potentially modified region was amplified using two sequential PCRs using the Illumina corporation technology driven by the NextSeq 500 Mid Output Kit v2.5 (300 cycles); Ref: 20024905. The first PCR corresponds to the specific amplification of the quoted region using the following forward and reverse PCR primers.

| **Name** | **Sequence** | **SEQ ID NO:** |
|---|---|---|
| GS1_F primer | ACACTCTTTCCCTACACGACGCTCTTCCGATCTTGGCCTT ATTCTAAAGGTCAACA | 32 |
| GS1_R primer | GTGACTGGAGTTCAGACGTGTGCTCTTCCGATCTCATTCT CCTCCCGCATGG | 33 |

These primers were synthetically made and contained at the 5' end the generic adaptors F-adaptor (Rd1SP, 33 nucleotides) and R-adaptor (Rd2SP, 34 nucleotides). The theoretical size of the amplified sequence was of 399 nucleotides if no deletion was observed.

In a second PCR, this fragment was re-amplified in order to be indexed using T5 i5 indexed primer and T7 i7 indexed primer and based on the generic sequences Rd1SP and Rd2SP described above. Indexation permitted the mixing of multiple fragments.

After sequencing, deletion within the GS genomic sequences of the two alleles was verified, confirming the disruption of the two GS gene alleles.

### Example 4: Antibody production assays

A trispecific antibody was produced using the double GS DHODH knock-out cells obtained in Example 3.

The expression vectors were designed, constructed and produced, at a concentration of 5 mg/ml. In these vectors, expression cassette and selection markers (either GS cDNA or DHODH G202A cDNAs) are bordered by ITRs (Inverted Terminal Repeats) allowing the use of piggybac transposon system for active integration of the expression cassettes and selection markers into the genome of CHO producing cells.

A plasmid for the mutated piggybac transposase (Yusa et al. (2011) Proc. Natl. Acad. Sci. USA 108:1531-1536) was co transfected to permit transient expression of the transposase.

The cell lines used were CHO 9E4_SP11 (disclosed in Example 1) and the knockout clones for DHODH and GS genes disclosed in Example 3.

CHO 9E4_SP11 was cultured in CDCHO Medium supplemented with 6 mM L-glutamine. Double KO clones were cultured in CDCHO medium supplemented with 6 mM L-Glutamine and 5 mM Uridine.

The cells to be transfected were cultivated in 25 mL working in 125 ml Erlenmeyer flasks at the beginning and amplified until the number of viable cells was sufficient to perform all the transfections.

One day before FectoPRO^{®} (Polyplus) transfection, the cells were diluted at 1.5×10⁶ cells/ml in CD CHO medium supplemented with 6 mM glutamine and 5 mM uridine.

On the day of transfection, 2.8 ml of diluted cell suspension were distributed in 24 DW plate at 1.1×10⁶ cells/ml in CD CHO complemented with 6 mM L-glutamine and 5 mM uridine. Following the manufacturer's recommended protocol, the FectoPRO^{®} reagent was vortexed for 5 s and spin down, before 6.2 µl per well were added to an empty 96 MTP well plate. In a second 96 MTP well plate 3.1 µg of DNA (90% of expression cassette DNA and 10% of transposase expression plasmid pBH6209) were diluted in 200 µl of CD CHO medium and the diluted DNA in culture was then distributed into the pure FectoPRO^{®} reagent all at once, respectively for each well. The solution was homogenized immediately and incubated for 10 min at room temperature. The FectoPRO^{®}/DNA transfection mix was poured onto the prepared cells previously distributed in 24 well plates and the culture was incubated at 37°C, 190 rpm and 8% CO₂.

Twenty-four hours post electroporation (Day 1), the cells were counted with ViCell XR. The whole cell culture was centrifuged at 250 g, 10 min, 25°C and the medium was carefully aspirated. The pellet was resuspended with pre-warmed CD OPTiCHO medium supplemented with 30% of FeedB and 25 µM of teriflunomide for CHO 9E4 WT cells and without any selection pressure or glutamine/uridine addition for the double KO GS/DHODH cells.

At day 3 post transfection, the cells were counted with Invitrogen^{™} Countess^{™} apparatus. The whole cell culture was centrifuged at 250 g, 10 min, 25°C and the supernatant was carefully discarded. The pellet was resuspended with pre-warmed CD OPTiCHO medium supplemented with 30% of FeedB and 25 µM of teriflunomide for CHO 9E4 WT cells and without any selection pressure or glutamine/uridine addition for double KO GS/DHODH cells.

At day 14 post transfection, the totality of the cultures was centrifuged at 250 g, 10 min, 4°C, the supernatant containing the produced protein of interest was filtered through a 0.22 µm PES filter and the antibody target titer was measured using Octet robot.

The table below resumes the transfection conditions for trispecific antibody.

| Expression vectors used for transfection | Cells | Selection marker | Day of transfection medium | Post-transfection selective medium |
|---|---|---|---|---|
| pVA4-00080/pVA4-0081 | DOUBLE KO1 | GS/DHODH | CD CHO + Uri + Gin | CD OPTiCHO + FeedB |
| pVA4-00080/pVA4-0081 | DOUBLE KO2 | GS/DHODH | CD CHO + Uri + Gin | CD OPTiCHO + FeedB |
| pVA4-00080/pVA4-0081 | DOUBLE KO3 | GS/DHODH | CD CHO + Uri + Gin | CD OPTiCHO + FeedB |
| pVA4-00080/pVA4-0081 | DOUBLE KO4 | GS/DHODH | CD CHO + Uri + Gin | CD OPTiCHO + FeedB |
| pVA4-00080/pVA4-0081 | DOUBLE KO5 | GS/DHODH | CD CHO + Uri + Gin | CD OPTiCHO + FeedB |
| pVA4-00080/pVA4-0081 | DOUBLR KO6 | GS/DHODH | CD CHO + Uri + Gin | CD OPTiCHO + FeedB |
| pVA4-00080/pVA4-0081 | DOUBLE KO7 | GS/DHODH | CD CHO + Uri + Gin | CD OPTiCHO + FeedB |
| pVA4-00080/pVA4-0081 | DOUBLE KO8 | GS/DHODH | CD CHO + Uri + Gin | CD OPTiCHO + FeedB |
| pVA4-00080/pVA4-0081 | DOUBLE KO9 | GS/DHODH | CD CHO + Uri + Gin | CD OPTiCHO + FeedB |
| pVA4-00080/pVA4-0081 | DOUBLE KO10 | GS/DHODH | CD CHO + Uri + Gin | CD OPTiCHO + FeedB |
| pVA4-00080/pVA4-0081 | DOUBLE KO11 | GS/DHODH | CD CHO + Uri + Gin | CD OPTiCHO + FeedB |
| pVA4-00080/pVA4-0081 | DOUBLE KO12 | GS/DHODH | CD CHO + Uri + Gin | CD OPTiCHO + FeedB |
| pVA4-00080/pVA4-0081 | DOUBLE KO13 | GS/DHODH | CD CHO + Uri + Gin | CD OPTiCHO + FeedB |
| pVA4-00080/pVA4-0081 | DOUBLE KO14 | GS/DHODH | CD CHO + Uri + Gin | CD OPTiCHO + FeedB |
| pVA4-00080/pVA4-0081 | DOUBLE KO15 | GS/DHODH | CD CHO + Uri + Gin | CD OPTiCHO + FeedB |
| pVA4-00080/pVA4-0081 | DOUBLE KO16 | GS/DHODH | CD CHO + Uri + Gin | CD OPTiCHO + FeedB |
| pVA4-00080/pVA4-0081 | DOUBLE KO17 | GS/DHODH | CD CHO + Uri + Gin | CD OPTiCHO + FeedB |
| pVA4-00080/pVA4-0081 | DOUBLE KO18 | GS/DHODH | CD CHO + Uri + Gin | CD OPTiCHO + FeedB |
| pVA4-00080/pVA4-0081 | DOUBLE KO19 | GS/DHODH | CD CHO + Uri + Gin | CD OPTiCHO + FeedB |
| pVA4-00080/pVA4-0081 | DOUBLE KO20 | GS/DHODH | CD CHO + Uri + Gin | CD OPTiCHO + FeedB |
| pVA4-00080/pVA4-0081 | 9E4WT | GS/DHODH | CD CHO + Gin | CD OPTiCHO + FeedB |
| pVA4-00080/pVA4-0081 | 9E4WT | GS/DHODH | CD CHO + Gin | CD OPTiCHO + FeedB |
| pVA4-00080/pVA4-0081 | 9E4WT | GS/DHODH | CD CHO + Gin | CD OPTiCHO + FeedB + MSX + TNF |
| pVA4-00080/pVA4-0081 | 9E4WT | GS/DHODH | CD CHO + Gin | CD OPTiCHO + FeedB + MSX + TNF |

| | | | | |
|---|---|---|---|---|
| Gin: glutamine, Uri: uridine, TNF: teriflunomide, MSX: L-methionine sulfoximine | | | | |

The following results were obtained.

| | **Number of wells** |
|---|---|
| Tested | **192** |
| Occupied | **96** |
| % of Occupied | 50 |
| Number of clones Gln sensitive | **45** |
| % of Gln sensitivity | 47 |

| | Viability of CHO cells at day 3 | | Viability of CHO cells at day 7 | |
|---|---|---|---|---|
| | Live cells (×10⁶/ml) | Viability (%) | Live cells (×10⁶/ml) | Viability (%) |
| DOUBLE KO1 | 1.75 | 87.01 | 6.56 | 97.17 |
| DOUBLE KO2 | 2.05 | 87.73 | 6.45 | 97.98 |
| DOUBLE KO3 | 2.7 | 88.75 | 7.57 | 97.77 |
| DOUBLE KO4 | 2.45 | 88.59 | 4.53 | 98.31 |
| DOUBLE KO5 | 2.64 | 86.26 | 6.3 | 94.85 |
| DOUBLE KO6 | 2.77 | 87.12 | 7.71 | 96.58 |
| DOUBLE KO7 | 2.8 | 84.65 | 7.62 | 96.17 |
| DOUBLE KO8 | 2.55 | 87.35 | 7.94 | 96.97 |
| DOUBLE KO9 | 2.45 | 86.18 | 7.42 | 96.7 |
| DOUBLE KO10 | 1.87 | 87.96 | 6.56 | 96.17 |
| DOUBLE KO11 | 2.4 | 83.81 | 7.39 | 95.83 |
| DOUBLE KO12 | 2.01 | 87.9 | 4.89 | 96.85 |
| DOUBLE KO13 | 2.53 | 88.21 | 6.07 | 97.8 |
| DOUBLE KO14 | 2.1 | 84.82 | 7.41 | 96.3 |
| DOUBLE KO15 | 2.17 | 85.85 | 7.03 | 97.82 |
| DOUBLE KO16 | 2.06 | 85.5 | 7.15 | 97.34 |
| DOUBLE KO17 | 2.6 | 85.95 | 8.06 | 97.23 |
| DOUBLE KO18 | 2.4 | 88.15 | 6.96 | 97.38 |
| DOUBLE KO19 | 2.5 | 85.82 | 7.5 | 96.04 |
| DOUBLE KO20 | 2.6 | 88.34 | 7.43 | 98.28 |
| 9E4 WT + MSX+TNF | 1.64 | 68.3 | 4.4 | 91.3 |

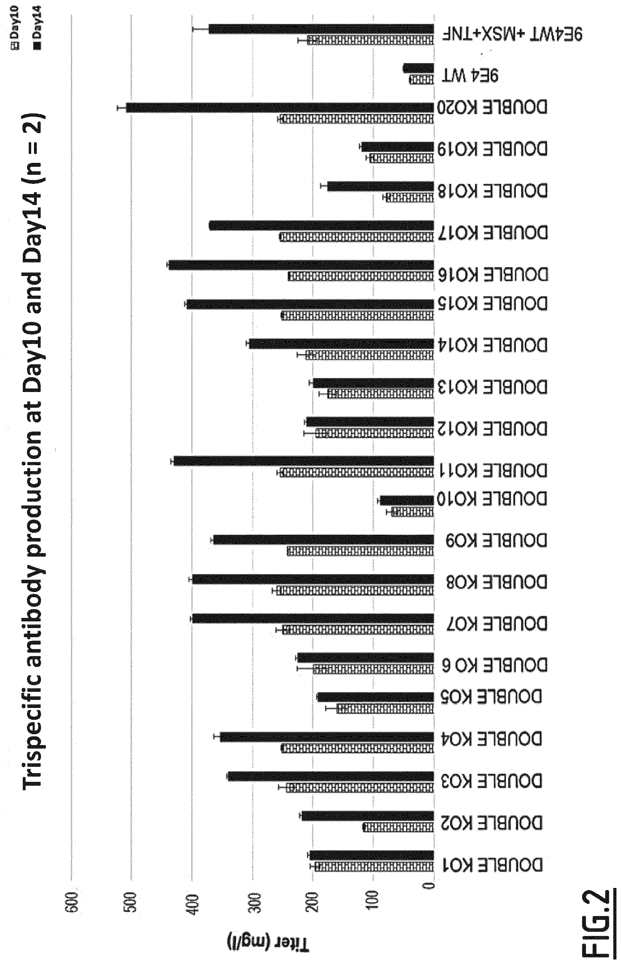

Trispecific antibody production at days 10 and 14 is shown on **Figure 2****.**

As shown on **Figure 2****,** ten clones displayed good production without any selection pressure Teriflunomide/L-methionine sulfoximine or glutamine/uridine addition.

These double KO clones produced the trispecific antibody in the same range as the prior art selection system, *i.e.* between 0.3 g/l and 0.5 g/l, with a particular high production by the clone double KO20.

### Example 5: Phenotypic and genotypic analysis of double KO20 clone

As the clone double KO20, was the most promising, further analysis of this clone was performed.

### A-Phenotypic analysis

Inactivation of the gene encoding GS in this clone was further investigated by Western Blot.

CHO culture media for double KO GS/DHODH was prepared. It was a CD-CHO medium with 6mM glutamine and 5mM Uridine pre warmed at 37°C for approximately 30 min by placing in a 37°C incubator. For preparation of CHO cells, the cells were counted with ViCell XR, and diluted in pre-warmed complemented medium. Then cells were incubated in a 37°C incubator containing a humidified atmosphere of 8% CO₂ in air on a 5mm orbital shaker platform rotating at 115 rpm.

Ten million cells were harvested at 800g during 5 minutes in a 15 ml tube. The supernatant was removed and the pellet was resuspended with 1 ml of M-PER buffer according to the supplier's recommendations. After 30 min of incubation at 4°C, a new centrifugation was performed during 15 min at 12000 rpm in a 1.5mL Eppendorf tube. Then 800µL of supernatant was collected for Western Blot assays. The protein concentration of the supernatant was determined by BCA assay.

4.4 µg of the sample was loaded on SDS-PAGE. Western blot analysis was performed by protein transfer on nitrocellulose membrane using the iBlot^{®} gel transfer tool (Life Technologies). The nitrocellulose membrane was blocked with a mix of BSA and 5% milk powder in PBS-T buffer (12 mM sodium phosphate, pH 7.4, 137 mM NaCl, 2.7 mM KCI, 0.05% Tween^{®}20). The nitrocellulose membrane was then incubated with the primary antibody (anti glutamine synthetase from Sigma (G2781) for 1 hour at room temperature, then incubated with an HRP conjugated anti-rabbit secondary antibody for 1 hour at room temperature. Proteins containing the epitope were then visualized using the ECL reagent for western blot detection.

A band corresponding to the expected molecular weight for glutamine synthetase (~ 45 kDa) is observed for the control sample (wild type CHO) and not for the sample from double KO20 clone. This western blotting analysis thus demonstrated the absence of glutamine synthetase in the sample from the double KO20 clone. This result confirms the complete inactivation of the endogenous glutamine synthetase (GS) gene in this productive clone.

In a further analysis, genotype of this clone was further investigated. The aim is to identify the mutation responsible of the KO in the genomic sequence of GS. The target sequence of the CRISPR cleavage was located in the vicinity of exon 6 of the GS gene, particularly, in the intron between exon 6 and exon 7.

In order to amplify the sequence of interest, PCR primers were designed from either side of the intron, on the bordering exons.

| **Amplification primers** | **Name** | **Targeted GS gene site** | **Sequence** | **SEQ ID NO:** |
|---|---|---|---|---|
| Forward | PCR_GS_for1 | 3'end of exon 6 | acctttgaccccaagc | 35 |
| Reverse | PCR_GS_rev2 | 3'end of exon 7 | cgtcgccagtctcattg | 36 |

Genomic DNA was extracted from the double KO20 clone as described in example 3, part C. It permits to obtain 98µl of DNA at 150ng/µl.
The PCR was performed and PCR products were controlled on agarose gel electrophoresis. The expected molecular weight was 879 bp. The inventors observed a slight band around the expected molecular weight and a major smaller band (around 400-500 bp) indicating several populations of gDNA in the sample from double KO20 clone. Thus, PCR amplifications performed allowed to obtain 2 types of amplicons.

The amplicons obtained were then cloned into a shuttle vector. After bacterial transformation, some colonies have been cultivated, and plasmid DNA was then purified by minipreparations. After DNA minipreparation, the plasmids containing the amplicons were sequenced, with primers bordering the insertion site in the shuttle vector.
The obtained sequences were aligned with the theoretical complete sequence of the GS gene.

The largest amplicons correspond to the presence of the intron between exons 6 and 7, wherein this intron is deleted of around 39bp.
The shorter amplicons correspond to the complete deletion of the intron between exons 6 and 7.

Regarding these analyses, the inventors concluded that the double KO20 clone was KO for GS with 2 populations of gDNA (one with full deletion of the intron and one with a 39bp deletion in the intron). These modifications in the gene encoding GS lead to the full inactivation of GS expression.

## Claims

1. A cell line comprising:
- an endogenous dehydroorotate dehydrogenase (DHODH) gene which is partially or fully inactivated, and
- an endogenous glutamine synthetase (GS) gene which is partially or fully inactivated.

2. The cell line according to claim 1, which is a Chinese Hamster Ovary (CHO) cell line.

3. The cell line according to claim 1 or 2, wherein the cell line is produced by:
(a1) inactivating the endogenous DHODH gene in a cell comprising an endogenous DHODH gene and an endogenous GS gene,
(b1) culturing the cell in a culture medium comprising uridine under conditions suitable for generating a cell line in which the endogenous DHODH gene is partially or fully inactivated,
(c1) recovering said cell wherein the endogenous DHODH gene is partially or fully inactivated,
(d1) inactivating the endogenous GS gene in said cell, and
(e1) culturing said cell in a culture medium comprising glutamine under conditions suitable for generating a cell line in which the endogenous GS gene is partially or fully inactivate.

4. The cell line according to claim 1 or 2, wherein the cell line is produced by:
(a2) providing a cell comprising an endogenous GS gene, and an endogenous DHODH gene which is partially or fully inactivated,
(b2) inactivating the endogenous GS gene is said cell, and
(c2) culturing the cell in a culture medium comprising glutamine under conditions suitable for generating a cell line in which the endogenous GS gene is partially or fully inactivated.

5. The cell line according to claim 1 or 2, wherein the cell line is produced by:
(a3) providing a cell comprising and endogenous DHODH gene, and an endogenous GS gene which is partially or fully inactivated,
(b3) inactivating the endogenous DHODH gene in said cell, and
(c3) culturing the cell in a culture medium comprising uridine under conditions suitable for generating a cell line in which the endogenous DHODH gene is partially or fully inactivated.

6. The cell line according to any one of claims 3 to 5, wherein the endogenous DHODH gene and/or the endogenous GS gene is inactivated by a gene-editing method.

7. The cell line according to claim 6, wherein the endogenous DHODH gene and/or the endogenous GS gene is inactivated by the CRISPR-Cas9 method.

8. The cell line according to any one of claims 1 to 7, wherein:
- one or more or all the alleles of the endogenous DHODH gene are partially or fully inactivated, and/or
- one or more or all the alleles of the endogenous GS gene are partially or fully inactivated.

9. The cell line according to any one of claims 1 to 8, wherein the cell line further comprises:
- an expression vector comprising a nucleotide sequence (i) encoding an exogenous mammalian DHODH and at least one expression cassette for expressing a recombinant protein (I), wherein said exogenous DHODH comprises a sequence at least 60% identical to the sequence SEQ ID NO: 2 or to the sequence SEQ ID NO: 4, and
- an expression vector comprising a nucleotide sequence (ii) encoding an exogenous mammalian GS and at least one expression cassette for expressing a recombinant protein (II), wherein said exogenous GS comprises a sequence at least 94.5% identical to the sequence SEQ ID NO: 6 or to the sequence SEQ ID NO: 8.

10. An expression system comprising:
(A) the cell line according to any one of claims 1 to 9,
(B) an expression vector comprising a nucleotide sequence (i) encoding a mammalian DHODH and at least one expression cassette for expressing a recombinant protein (I), wherein said DHODH comprises a sequence at least 60% identical to the sequence SEQ ID NO: 2 or to the sequence SEQ ID NO: 4, and
(C) an expression vector comprising a nucleotide sequence (ii) encoding a mammalian GS ad at least one expression cassette for expressing a recombinant protein (II), wherein the GS comprises a sequence at least 94.5% identical to the sequence SEQ ID NO: 6 or the sequence SEQ ID NO: 8.

11. The cell line according to claim 9 or the expression system according to claim 10, wherein said nucleotide sequence (i) comprises the sequence of SEQ ID NO: 1 or the sequence of SEQ ID NO: 3.

12. The cell line according to claim 9 or 11 or the expression system according to claim 10 or 11, wherein said nucleotide sequence (ii) comprises the sequence of SEQ ID NO: 5 or the sequence of SEQ ID NO: 7.

13. The cell line according to any one of claims 9, 11 and 12 or the expression system according to any one of claims 10, 11 and 12, wherein said recombinant proteins (I) and (II) are distinct arms of a monoclonal antibody.

14. The cell line according to any one of claims 9 and 11-13 or the expression system according to any one of claims 10-13, wherein said vectors comprise a first expression cassette suitable for cloning of an antibody light chain, and a second expression cassette suitable for cloning of an antibody heavy chain.

15. A kit comprising:
- the cell line according to any one of claims 9 and 11-14 or the expression system according to any one of claims 10-14, and
- a culture medium devoid of uridine and of glutamine, in particular further devoid of DHODH inhibitor and of GS inhibitor.

16. An *in vitro* method of producing recombinant proteins comprising the steps of:
A)
a1) providing a cell line according to any one of claims 9 and 11-14;
or
a2) providing a cell line according to any one of claims 1 to 8, and
a2') introducing expression vectors as defined in any one of claims 10 to 14 into the cell line provided in step a2);
or
a3) providing a cell line comprising an endogenous DHODH gene and an endogenous GS gene,
a3') partially or fully inactivating the endogenous DHODH gene and the endogenous GS gene in the cell line provided in step a3), and
a3") introducing expression vectors as defined in any one of claims 10 to 14 into the cell line comprising a partially or fully inactivated endogenous DHODH gene and a partially or fully inactivated GS gene obtained in step a3');
or
a4) providing a cell comprising an endogenous GS gene, and an endogenous DHODH gene which is partially or fully inactivated,
a4') partially or fully inactivating the endogenous GS gene in the cell line provided in step a4), and
a4") introducing expression vectors as defined in any one of claims 10 to 14 into the cell line comprising a partially or fully inactivated endogenous DHODH gene and a partially or fully inactivated GS gene obtained in step a4');
or
a5) providing a cell comprising an endogenous DHODH gene, and an endogenous GS gene which is partially or fully inactivated,
a5') partially or fully inactivating the endogenous DHODH gene in the cell line provided in step a5), and
a5") introducing expression vectors as defined in any one of claims 10 to 14 into the cell line comprising a partially or fully inactivated endogenous DHODH gene and a partially or fully inactivated GS gene obtained in step a5');
B) culturing said cell line under conditions suitable for production of the recombinant proteins; and
C) isolating and/or purifying said recombinant proteins.

17. The method according to claim 16, wherein step B) is conducted in a culture medium devoid of uridine and of glutamine, in particular further devoid of DHODH inhibitor and of GS inhibitor.

18. The method according to claim 16 or 17, further comprising a step D) of formulating said recombinant proteins into a pharmaceutical composition.

19. Use of a cell line according to any one of claims 9 and 11-14, an expression system according to any one of claims 10 to 14, or a kit according to claim 15, for producing recombinant proteins.

20. The use according to claim 19, wherein the cell line, the expression system or the kit is used in combination with a culture medium devoid of uridine and of glutamine, in particular in the absence of DHODH inhibitor and of GS inhibitor.

## Patentansprüche

1. Zelllinie, umfassend:
- ein endogenes Dihydroorotatdehydrogenase (DHODH)-Gen, das teilweise oder vollständig inaktiviert ist, und
- ein endogenes Glutaminsynthetase (GS)-Gen, das teilweise oder vollständig inaktiviert ist.

2. Zelllinie gemäß Anspruch 1, welche eine China-Hamster-Ovarial-Zelllinie (CHO-Zelllinie) ist.

3. Zelllinie gemäß Anspruch 1 oder 2, wobei die Zelllinie hergestellt wird mittels:
(a1) Inaktivieren des endogenen DHODH-Gens in einer Zelle, die ein endogenes DHODH-Gen und ein endogenes GS-Gen umfasst,
(b1) Kultivieren der Zelle in einem Uridin umfassenden Kulturmedium unter Bedingungen, geeignet zum Erzeugen einer Zelllinie, in der das endogene DHODH-Gen teilweise oder vollständig inaktiviert ist,
(c1) Rückgewinnen der Zelle, in der das endogene DHODH-Gen teilweise oder vollständig inaktiviert ist,
(d1) Inaktivieren des endogenen GS-Gens in der Zelle, und
(e1) Kultivieren der Zelle in einem Glutamin umfassenden Kulturmedium unter Bedingungen, geeignet zum Erzeugen einer Zelllinie, in der das endogene GS-Gen teilweise oder vollständig inaktiviert ist.

4. Zelllinie gemäß Anspruch 1 oder 2, wobei die Zelllinie hergestellt wird mittels:
(a2) Bereitstellen einer Zelle, umfassend ein endogenes GS-Gen und ein endogenes DHODH-Gen, das teilweise oder vollständig inaktiviert ist,
(b2) Inaktivieren des endogenen GS-Gens in der Zelle, und
(c2) Kultivieren der Zelle in einem Glutamin umfassenden Kulturmedium unter Bedingungen, geeignet zum Erzeugen einer Zelllinie, in der das endogene GS-Gen teilweise oder vollständig inaktiviert ist.

5. Zelllinie gemäß Anspruch 1 oder 2, wobei die Zelllinie hergestellt wird mittels:
(a3) Bereitstellen einer Zelle, umfassend ein endogenes DHODH-Gen und ein endogenes GS-Gen, das teilweise oder vollständig inaktiviert ist,
(b3) Inaktivieren des endogenen DHODH-Gens in der Zelle, und
(c3) Kultivieren der Zelle in einem Uridin umfassenden Kulturmedium unter Bedingungen, geeignet zum Erzeugen einer Zelllinie, in der das endogene DHODH-Gen teilweise oder vollständig inaktiviert ist.

6. Zelllinie gemäß einem der Ansprüche 3 bis 5, wobei das endogene DHODH-Gen und/oder das endogene GS-Gen durch ein Geneditierungsverfahren inaktiviert wird.

7. Zelllinie gemäß Anspruch 6, wobei das endogene DHODH-Gen und/oder das endogene GS-Gen durch das CRISPR-Cas9-Verfahren inaktiviert wird.

8. Zelllinie gemäß einem der Ansprüche 1 bis 7, wobei:
- eines oder mehrere oder alle der Allele des endogenen DHODH-Gens teilweise oder vollständig inaktiviert sind, und/oder
- eines oder mehrere oder alle der Allele des endogenen GS-Gens teilweise oder vollständig inaktiviert sind.

9. Zelllinie gemäß einem der Ansprüche 1 bis 8, wobei die Zelllinie ferner umfasst:
- einen Expressionsvektor, umfassend eine Nukleotidsequenz (i), codierend eine exogene Säuger-DHODH, und mindestens eine Expressionskassette zum Exprimieren eines rekombinanten Proteins (I), wobei die exogene DHODH eine Sequenz umfasst, die mindestens 60 % identisch zur Sequenz SEQ ID NO: 2 oder zur Sequenz SEQ ID NO: 4 ist, und
- einen Expressionsvektor, umfassend eine Nukleotidsequenz (ii), codierend eine exogene Säuger-GS, und mindestens eine Expressionskassette zum Exprimieren eines rekombinanten Proteins (II), wobei die exogene GS eine Sequenz umfasst, die mindestens 94,5 % identisch zur Sequenz SEQ ID NO: 6 oder zur Sequenz SEQ ID NO: 8 ist.

10. Expressionssystem, umfassend:
(A) die Zelllinie gemäß einem der Ansprüche 1 bis 9,
(B) einen Expressionsvektor, umfassend eine Nukleotidsequenz (i), codierend eine Säuger-DHODH, und mindestens eine Expressionskassette zum Exprimieren eines rekombinanten Proteins (I), wobei die DHODH eine Sequenz umfasst, die mindestens 60 % identisch zur Sequenz SEQ ID NO: 2 oder zur Sequenz SEQ ID NO: 4 ist, und
(C) einen Expressionsvektor, umfassend eine Nukleotidsequenz (ii), codierend eine Säuger-GS, und mindestens eine Expressionskassette zum Exprimieren eines rekombinanten Proteins (II), wobei die GS eine Sequenz umfasst, die mindestens 94,5 % identisch zur Sequenz SEQ ID NO: 6 oder zur Sequenz SEQ ID NO: 8 ist.

11. Zelllinie gemäß Anspruch 9 oder Expressionssystem gemäß Anspruch 10, wobei die Nukleotidsequenz (i) die Sequenz von SEQ ID NO: 1 oder die Sequenz von SEQ ID NO: 3 umfasst.

12. Zelllinie gemäß Anspruch 9 oder 11 oder Expressionssystem gemäß Anspruch 10 oder 11, wobei die Nukleotidsequenz (ii) die Sequenz von SEQ ID NO: 5 oder die Sequenz von SEQ ID NO: 7 umfasst.

13. Zelllinie gemäß einem der Ansprüche 9, 11 und 12 oder Expressionssystem gemäß einem der Ansprüche 10, 11 und 12, wobei die rekombinanten Proteine (I) und (II) unterschiedliche Arme eines monoklonalen Antikörpers sind.

14. Zelllinie gemäß einem der Ansprüche 9 und 11-13 oder Expressionssystem gemäß einem der Ansprüche 10-13, wobei die Vektoren eine erste Expressionskassette, geeignet zum Klonieren einer Antikörperleichtkette, und eine zweite Expressionskassette, geeignet zum Klonieren einer Antikörperschwerkette, umfassen.

15. Kit, umfassend:
- die Zelllinie gemäß einem der Ansprüche 9 und 11-14 oder das Expressionssystem gemäß einem der Ansprüche 10-14, und
- ein Kulturmedium, das frei von Uridin und von Glutamin ist, des Weiteren insbesondere frei von DHODH-Inhibitor und von GS-Inhibitor ist.

16. In-vitro-Verfahren zur Herstellung rekombinanter Proteine, umfassend die folgenden Schritte:
A) a1) Bereitstellen einer Zelllinie gemäß einem der Ansprüche 9 und 11-14;
oder
a2) Bereitstellen einer Zelllinie gemäß einem der Ansprüche 1 bis 8, und
a2') Einbringen von Expressionsvektoren, wie in einem der Ansprüche 10 bis 14 definiert, in die im Schritt a2) bereitgestellte Zelllinie;
oder
a3) Bereitstellen einer Zelllinie, umfassend ein endogenes DHODH-Gen und ein endogenes GS-Gen,
a3') teilweises oder vollständiges Inaktivieren des endogenen DHODH-Gens und des endogenen GS-Gens in der im Schritt a3) bereitgestellten Zelllinie, und
a3") Einbringen von Expressionsvektoren, wie in einem der Ansprüche 10 bis 14 definiert, in die im Schritt a3') erhaltene Zelllinie, die ein teilweise oder vollständig inaktiviertes endogenes DHODH-Gen und ein teilweise oder vollständig inaktiviertes GS-Gen umfasst;
oder
a4) Bereitstellen einer Zelle, umfassend ein endogenes GS-Gen und ein endogenes DHODH-Gen, das teilweise oder vollständig inaktiviert ist,
a4') teilweises oder vollständiges Inaktivieren des endogenen GS-Gens in der im Schritt a4) bereitgestellten Zelllinie, und
a4") Einbringen von Expressionsvektoren, wie in einem der Ansprüche 10 bis 14 definiert, in die im Schritt a4') erhaltene Zelllinie, die ein teilweise oder vollständig inaktiviertes endogenes DHODH-Gen und ein teilweise oder vollständig inaktiviertes GS-Gen umfasst;
oder
a5) Bereitstellen einer Zelle, umfassend ein endogenes DHODH-Gen und ein endogenes GS-Gen, das teilweise oder vollständig inaktiviert ist,
a5') teilweises oder vollständiges Inaktivieren des endogenen DHODH-Gens in der im Schritt a5) bereitgestellten Zelllinie, und
a5") Einbringen von Expressionsvektoren, wie in einem der Ansprüche 10 bis 14 definiert, in die im Schritt a5') erhaltene Zelllinie, die ein teilweise oder vollständig inaktiviertes endogenes DHODH-Gen und ein teilweise oder vollständig inaktiviertes GS-Gen umfasst;
B) Kultivieren der Zelllinie unter für die Produktion der rekombinanten Proteine geeigneten Bedingungen; und
C) Isolieren und/oder Reinigen der rekombinanten Proteine.

17. Verfahren gemäß Anspruch 16, wobei der Schritt B) durchgeführt wird in einem Kulturmedium, das frei von Uridin und von Glutamin ist, des Weiteren insbesondere frei von DHODH-Inhibitor und von GS-Inhibitor ist.

18. Verfahren nach Anspruch 16 oder 17, ferner umfassend einen Schritt D) des Formulierens der rekombinanten Proteine in eine pharmazeutische Zusammensetzung.

19. Verwendung einer Zelllinie gemäß einem der Ansprüche 9 und 11-14, eines Expressionssystems gemäß einem der Ansprüche 10 bis 14, oder eines Kits gemäß Anspruch 15, zum Produzieren von rekombinanten Proteinen.

20. Verwendung gemäß Anspruch 19, wobei die Zelllinie, das Expressionssystem oder das Kit in Kombination mit einem Kulturmedium, das frei von Uridin und von Glutamin ist, insbesondere in Abwesenheit von DHODH-Inhibitor und von GS-Inhibitor, verwendet wird.

## Revendications

1. Lignée cellulaire comprenant :
- un gène endogène de la dihydroorotate déshydrogénase (DHODH) qui est partiellement ou totalement inactivé, et
- un gène endogène de la glutamine synthétase (GS) qui est partiellement ou totalement inactivé.

2. Lignée cellulaire selon la revendication 1, qui est une lignée cellulaire d'ovaire de hamster chinois (CHO).

3. Lignée cellulaire selon la revendication 1 ou 2, dans laquelle la lignée cellulaire est produite par :
(a1) inactivation du gène DHODH endogène dans une cellule comprenant un gène DHODH endogène et un gène GS endogène,
(b1) culture de la cellule dans un milieu de culture comprenant de l'uridine dans des conditions appropriées pour générer une lignée cellulaire dans laquelle le gène DHODH endogène est partiellement ou totalement inactivé,
(c1) récupération de ladite cellule dans laquelle le gène DHODH endogène est partiellement ou totalement inactivé,
(d1) inactivation du gène GS endogène dans ladite cellule, et
(e1) culture de ladite cellule dans un milieu de culture comprenant de la glutamine dans des conditions appropriées pour générer une lignée cellulaire dans laquelle le gène GS endogène est partiellement ou totalement inactivé.

4. Lignée cellulaire selon la revendication 1 ou 2, dans laquelle la lignée cellulaire est produite par :
(a2) fourniture d'une cellule comprenant un gène GS endogène, et un gène DHODH endogène qui est partiellement ou totalement inactivé,
(b2) inactivation du gène GS endogène dans ladite cellule, et
(c2) culture de la cellule dans un milieu de culture comprenant de la glutamine dans des conditions appropriées pour générer une lignée cellulaire dans laquelle le gène GS endogène est partiellement ou totalement inactivé.

5. Lignée cellulaire selon la revendication 1 ou 2, dans laquelle la lignée cellulaire est produite par :
(a3) fourniture d'une cellule comprenant un gène DHODH endogène, et un gène GS endogène qui est partiellement ou totalement inactivé,
(b3) inactivation du gène DHODH endogène dans ladite cellule, et
(c3) culture de la cellule dans un milieu de culture comprenant de l'uridine dans des conditions appropriées pour générer une lignée cellulaire dans laquelle le gène DHODH endogène est partiellement ou totalement inactivé.

6. Lignée cellulaire selon l'une quelconque des revendications 3 à 5, dans laquelle le gène DHODH endogène et/ou le gène GS endogène est inactivé par un procédé d'édition de gène.

7. Lignée cellulaire selon la revendication 6, dans laquelle le gène DHODH endogène et/ou le gène GS endogène est inactivé par le procédé CRISPR-Cas9.

8. Lignée cellulaire selon l'une quelconque des revendications 1 à 7, dans laquelle :
- un ou plusieurs ou tous les allèles du gène DHODH endogène sont partiellement ou totalement inactivés, et/ou
- un ou plusieurs ou tous les allèles du gène GS endogène sont partiellement ou totalement inactivés.

9. Lignée cellulaire selon l'une quelconque des revendications 1 à 8, dans laquelle la lignée cellulaire comprend en outre :
- un vecteur d'expression comprenant une séquence nucléotidique (i) codant pour une DHODH mammifère exogène et au moins une cassette d'expression pour exprimer une protéine recombinante (I), dans laquelle ladite DHODH exogène comprend une séquence au moins 60 % identique à la séquence SEQ ID NO : 2 ou à la séquence SEQ ID NO : 4, et
- un vecteur d'expression comprenant une séquence nucléotidique (ii) codant pour une GS mammifère exogène et au moins une cassette d'expression pour exprimer une protéine recombinante (II), dans laquelle ladite GS exogène comprend une séquence au moins 94,5 % identique à la séquence SEQ ID NO : 6 ou à la séquence SEQ ID NO : 8.

10. Système d'expression comprenant :
(A) la lignée cellulaire selon l'une quelconque des revendications 1 à 9,
(B) un vecteur d'expression comprenant une séquence nucléotidique (i) codant pour une DHODH mammifère et au moins une cassette d'expression pour exprimer une protéine recombinante (I), dans lequel ladite DHODH comprend une séquence au moins 60 % identique à la séquence SEQ ID NO : 2 ou à la séquence SEQ ID NO : 4, et
(C) un vecteur d'expression comprenant une séquence nucléotidique (ii) codant pour une GS mammifère et au moins une cassette d'expression pour exprimer une protéine recombinante (II), dans lequel la GS comprend une séquence au moins 94,5 % identique à la séquence SEQ ID NO : 6 ou la séquence SEQ ID NO : 8.

11. Lignée cellulaire selon la revendication 9 ou système d'expression selon la revendication 10, ladite séquence nucléotidique (i) comprenant la séquence de SEQ ID NO : 1 ou la séquence de SEQ ID NO : 3.

12. Lignée cellulaire selon la revendication 9 ou 11 ou système d'expression selon la revendication 10 ou 11, ladite séquence nucléotidique (ii) comprenant la séquence de SEQ ID NO : 5 ou la séquence de SEQ ID NO : 7.

13. Lignée cellulaire selon l'une quelconque des revendications 9, 11 et 12 ou système d'expression selon l'une quelconque des revendications 10, 11 et 12, dans laquelle/lequel lesdites protéines recombinantes (I) et (II) sont des bras distincts d'un anticorps monoclonal.

14. Lignée cellulaire selon l'une quelconque des revendications 9 et 11-13 ou système d'expression selon l'une quelconque des revendications 10 à 13, dans laquelle/lequel lesdits vecteurs comprennent une première cassette d'expression appropriée pour le clonage d'une chaîne légère d'anticorps, et une seconde cassette d'expression appropriée pour le clonage d'une chaîne lourde d'anticorps.

15. Kit comprenant :
- la lignée cellulaire selon l'une quelconque des revendications 9 et 11-14 ou le système d'expression selon l'une quelconque des revendications 10 à 14, et
- un milieu de culture dépourvu d'uridine et de glutamine, en particulier en outre dépourvu d'inhibiteur de DHODH et d'inhibiteur de GS.

16. Procédé *in vitro* de production de protéines recombinantes comprenant les étapes de :
A) a1) fourniture d'une lignée cellulaire selon l'une quelconque des revendications 9 et 11 à 14 ;
ou
a2) fourniture d'une lignée cellulaire selon l'une quelconque des revendications 1 à 8, et
a2') introduction de vecteurs d'expression tels que définis dans l'une quelconque des revendications 10 à 14 dans la lignée cellulaire fournie à l'étape a2) ;
ou
a3) fourniture d'une lignée cellulaire comprenant un gène DHODH endogène et un gène GS endogène,
a3') inactivation partielle ou totale du gène DHODH endogène et du gène GS endogène dans la lignée cellulaire fournie à l'étape a3), et
a3") introduction de vecteurs d'expression tels que définis dans l'une quelconque des revendications 10 à 14 dans la lignée cellulaire comprenant un gène DHODH endogène partiellement ou totalement inactivé et un gène GS partiellement ou totalement inactivé obtenue à l'étape a3') ;
ou
a4) fourniture d'une cellule comprenant un gène GS endogène, et un gène DHODH endogène qui est partiellement ou totalement inactivé,
a4') inactivation partielle ou totale du gène GS endogène dans la lignée cellulaire fournie à l'étape a4), et
a4") introduction de vecteurs d'expression tels que définis dans l'une quelconque des revendications 10 à 14 dans la lignée cellulaire comprenant un gène DHODH endogène partiellement ou totalement inactivé et un gène GS partiellement ou totalement inactivé obtenue à l'étape a4') ;
ou
a5) fourniture d'une cellule comprenant un gène DHODH endogène, et un gène GS endogène qui est partiellement ou totalement inactivé,
a5') inactivation partielle ou totale du gène DHODH endogène dans la lignée cellulaire fournie à l'étape a5), et
a5") introduction de vecteurs d'expression tels que définis dans l'une quelconque des revendications 10 à 14 dans la lignée cellulaire comprenant un gène DHODH endogène partiellement ou totalement inactivé et un gène GS partiellement ou totalement inactivé obtenue à l'étape a5') ;
B) culture de ladite lignée cellulaire dans des conditions appropriées à la production des protéines recombinantes ; et
C) isolement et/ou purification desdites protéines recombinantes.

17. Procédé selon la revendication 16, dans lequel l'étape B) est conduite dans un milieu de culture dépourvu d'uridine et de glutamine, en particulier en outre dépourvu d'inhibiteur de DHODH et d'inhibiteur de GS.

18. Procédé selon la revendication 16 ou 17, comprenant en outre une étape D) de formulation desdites protéines recombinantes dans une composition pharmaceutique.

19. Utilisation d'une lignée cellulaire selon l'une quelconque des revendications 9 et 11 à 14, d'un système d'expression selon l'une quelconque des revendications 10 à 14, ou d'un kit selon la revendication 15, pour la production de protéines recombinantes.

20. Utilisation selon la revendication 19, dans laquelle la lignée cellulaire, le système d'expression ou le kit est utilisé(e) en combinaison avec un milieu de culture dépourvu d'uridine et de glutamine, en particulier en l'absence d'inhibiteur de DHODH et d'inhibiteur de GS.
